# EUROPEAN PATENT APPLICATION

(11) **EP 4 079 250 A1**
(43) Date of publication of application: **26.10.2022**
(21) Application number: 22168993.8
(22) Date of filing: 20.04.2022
(51) Int. Cl.: A61B 34/30

(54) **SURGICAL INSTRUMENT ADAPTOR AND SURGERY ASSIST ROBOT**

(30) Priority: 20.04.2021 JP 2021071376
(71) Applicant: Medicaroid Corporation, Kobe-shi, Hyogo 650-0047 (JP)
(72) Inventor: AGO, Kenji, Hyogo, 650-0047 (JP); SUGA, Kazunori, Hyogo, 650-0047 (JP); HORITA, Shiro, Hyogo, 651-0083 (JP)
(74) Representative: Müller Hoffmann & Partner

(57) **Abstract**

A surgical instrument adaptor according to one or more embodiments for connecting a manual surgical instrument to a robot arm to operate the manual surgical instrument includes: an interface portion including a rotation member to be driven to rotate by a driving force transmitted from a driving part provided at the robot arm; a driven part including a pressing portion configured to press an operation portion of the manual surgical instrument; an elongate element comprising a wire or a cable connecting the rotation member and the driven part and configured to transmit the driving force from the rotation member to the driven part; and a guide tube that guides the elongate element.

## Description

### TECHNICAL FIELD

The invention relates to a surgical instrument adaptor and a surgery assist robot.

### BACKGROUND ART

In a related art, there is known a surgery assist robot to which a surgical tool is attached.

U.S. Patent Application Publication No. 2012/0211546 (hereinafter, may be simply referred to as Patent Document 1) discloses a surgery assist robot including a plurality of robotic manipulators. Each of the plurality of robotic manipulators is configured as an articulated robot. To the plurality of robotic manipulators, surgical tools are respectively attached.

In the surgery assist robot disclosed in Patent Document 1, each of the surgical tools includes a tool attachment portion for attaching the surgical tool to the robot manipulator. The tool attachment portion is provided with rotation members. The rotation members of the tool attachment portion are driven to rotate by driving parts (drivers) provided in the robot manipulator. By the rotational forces of the rotation members, the surgical tool (e.g., end cutter) or the like is driven. Although not specified in Patent Document 1, in general, the surgical tool that is to be attached to the robot manipulator is manufactured as a dedicated product compatible with (dedicated for) a mechanism (e.g., the drivers) or the like of the robot manipulator.

### SUMMARY

However, in the surgery assist robot as described above, since the surgical tool that is to be attached to the robot manipulator is the dedicated product compatible with the mechanism or the like of the robot manipulator, there may be a problem that existing manual surgical instruments (existing manual surgical tools), owned by the hospital or the like and manually operated by doctors or the like, cannot be operated by the robot manipulator of the surgery assist robot.

An object of the invention may be to provide a surgical instrument adaptor and a surgery assist robot that is capable of operating an existing manual surgical instrument without using a surgical instrument dedicated for the surgery assist robot.

A first aspect of the invention is a surgical instrument adaptor for connecting a manual surgical instrument to a robot arm such that the manual surgical instrument attached thereto is operable by the robot arm. The surgical instrument adaptor includes: an interface portion; a driven part, an elongate element comprising a wire or cable; and a guide tube. The interface portion includes a rotation member to be driven to rotate by a driving force transmitted from a driving part provided at the robot arm. The driven part includes a pressing portion to press an operation portion of the manual surgical instrument. The elongate element is connected to the rotation member and the driven part so as to transmit a driving force from the rotation member to the driven part. The guide tube guides the elongate element.

As described above, the surgical instrument adaptor according to the first aspect includes the interface portion that includes: the rotation member to be driven to rotate by the driving force transmitted from the driving part provided at the robot arm; and the driven part including the pressing portion to press the operation portion of the manual surgical instrument.

According to the first aspect described above, the operation portion of the manual surgical instrument can be operated (pressed) by driving (moving) the pressing portion of the driven part by the driving force of the driver provided at the robot arm. As a result, the existing manual surgical instrument can be operated without using a surgical instrument dedicated for a surgery assist robot. Further, since the first aspect described above is provided with the guide tube that guides the elongate element composed of the wire or the cable for transmitting the driving force from the rotation member to the driven part, even when the arrangement (layout) of the driven part is changed according to the position of the operation portion of the manual surgical instrument, the elongate element is guided by the guide tube to the driven part whose arrangement has been changed. Accordingly, even when the arrangement (layout) of the driven part is changed, the driven part can be driven by the elongate element guided by the guide tube. As a result, it is possible to flexibly respond to changes in the layout of the driven part.

A second aspect of the invention is a surgery assist robot that includes a robot arm including a driving part and a surgical instrument adaptor for attaching a manual surgical instrument to the robot arm such that the manual surgical instrument attached thereto is operable by the robot arm. The surgical instrument adaptor includes: an interface portion; a driven part, an elongate element comprising a wire or cable; and a guide tube. The interface portion includes a rotation member to be driven to rotate by a driving force transmitted from the driving part of the robot arm. The driven part includes a pressing portion to press an operation portion of the manual surgical instrument. The elongate element is connected to the rotation member and the driven part so as to transmit a driving force from the rotation member to the driven part. The guide tube guides the elongate element.

As described above, the surgery assist robot according to the second aspect includes the interface portion that includes: the rotation member to be driven to rotate by the driving force transmitted from the driving part provided at the robot arm; and the driven part including the pressing portion to press the operation portion of the manual surgical instrument. Accordingly, the operation portion of the manual surgical instrument can be operated (pressed) by driving (moving) the pressing portion of the driven part by the driving force of the driving part provided at the robot arm. As a result, it is possible to provide the surgery assist robot that can operate the existing manual surgical instrument without using a robotic surgical instrument dedicated for the surgery assist robot. Further, since the second aspect described above is provided with the guide tube that guides the elongate element composed of the wire or the cable for transmitting the driving force from the rotation member to the driven part, even when the arrangement (layout) of the driven part is changed according to the position of the operation portion of the manual surgical instrument, the elongate element is guided by the guide tube to the driven part whose arrangement has been changed. Therefore, it is possible to provide a surgery assist robot capable of driving the driven unit by the elongate element guided by the guide tube, even when the arrangement (layout) of the driven unit is changed. As a result, it is possible to flexibly respond to changes in the layout of the driven part.

According to the invention, it is possible to operate an existing manual surgical instrument without using a surgical instrument dedicated for a surgery assist robot.

### BRIEF DESCRIPTION OF THE DRAWINGS

FIG. 1 is a diagram illustrating a view of a configuration of a surgical operation system according to a first embodiment;
FIG. 2 is a diagram illustrating a view of a configuration of a medical manipulator according to a first embodiment;
FIG. 3 is a diagram illustrating a view of a configuration of an operation handle according to a first embodiment;
FIG. 4 is a diagram illustrating a view of foot pedals according to a first embodiment;
FIG. 5 is a diagram illustrating a view of a configuration of a robot arm of the medical manipulator according to a first embodiment;
FIG. 6 is a diagram illustrating a view of forceps;
FIG. 7 is a diagram illustrating a perspective view of a configuration of an operation unit of the medical manipulator according to a first embodiment;
FIG. 8 is a diagram illustrating a view of an endoscope;
FIG. 9 is a diagram illustrating a view of a pivot position setting device;
FIG. 10 is a diagram illustrating a view for explaining translational movements of the robot arm;
FIG. 11 is a diagram illustrating a view for explaining rotational movements of the robot arm;
FIG. 12 is a block diagram of a configuration of a control unit of the medical manipulator according to a first embodiment;
FIG. 13 is a diagram illustrating a perspective view of a state where an adaptor and a medical instrument (dedicated surgical instrument) are detached from driving parts of the robot arm according to a first embodiment;
FIG. 14 is a diagram illustrating a perspective view of the adaptor and the dedicated surgical instrument as seen from the Y2 side according to a first embodiment;
FIG. 15 is a diagram illustrating a view of a manual surgical instrument;
FIG. 16 is a diagram illustrating a view of a state where the manual surgical instrument is attached to a surgical instrument adaptor;
FIG. 17 is a diagram illustrating a view of plural driven parts;
FIG. 18 is a diagram illustrating a perspective view of an interface portion;
FIG. 19 is a diagram illustrating a view of the plural driven parts with a frame being omitted.
FIG. 20 is a diagram illustrating another view of the plural driven parts with the frame being omitted.
FIG. 21 is a diagram illustrating a perspective view of the driven part;
FIG. 22 is a diagram illustrating another perspective view of the driven part;
FIG. 23 is a diagram illustrating still another perspective view of the driven part;
FIG. 24 is a diagram illustrating an external view of the surgical instrument adaptor according to a first embodiment;
FIG. 25 is a diagram illustrating another external view of the surgical instrument adaptor according to a first embodiment;
FIG. 26 is a diagram illustrating a view of a guide tube;
FIG. 27 is a diagram illustrating a sectional view of the guide tube;
FIG. 28A is a diagram for explaining an operation of attaching the manual surgical instrument to the surgical instrument adaptor, which illustrating a view of a state before the manual surgical instrument is attached;
FIG. 28B is a diagram for explaining an operation of attaching the manual surgical instrument to the surgical instrument adaptor, which illustrates a view of a state in which the manual surgical instrument is inclined and inserted in the surgical instrument adaptor;
FIG. 28C is a diagram for explaining an operation of attaching the manual surgical instrument to the surgical instrument adaptor, which illustrates a view of a state in which the inclination of the manual surgical instrument is restored;
FIG. 28D is a diagram for explaining an operation of attaching the manual surgical instrument to the surgical instrument adaptor, which illustrates a view of a state in which the attachment of the manual surgical instrument is completed; and
FIG. 29 is a diagram illustrating a perspective view of a surgical instrument adaptor according to a second embodiment.

### DETAILED DESCRIPTION

Hereinafter, embodiments of the present invention will be described with reference to the drawings.

### (First Embodiment)

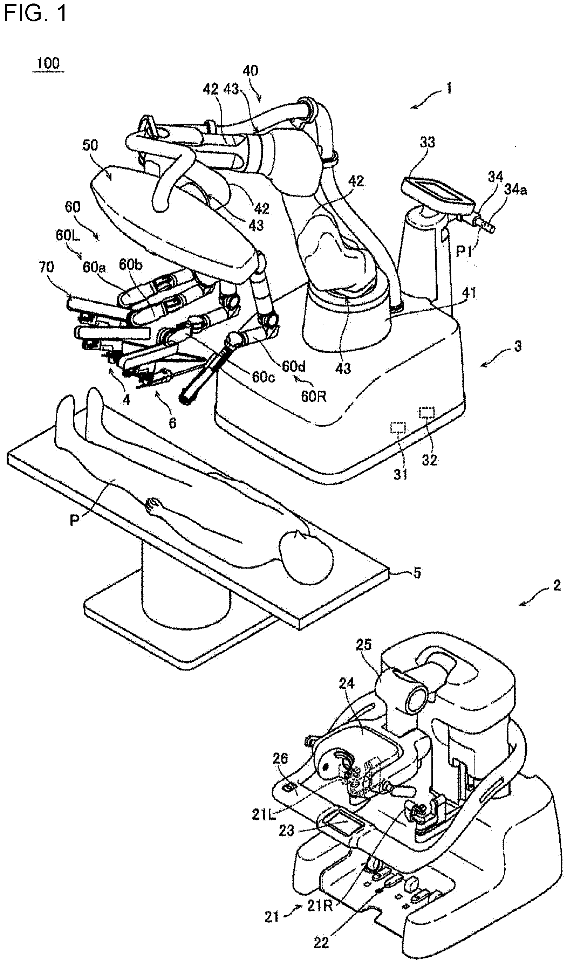

A configuration of a surgical operation system 100 according to a first embodiment is described with reference to FIGS. 1 to 28D. The surgical operation system 100 includes a medical manipulator 1 serving as a patient-side apparatus and a remote control apparatus 2 serving as an operator-side apparatus to operate the medical manipulator 1. The medical manipulator 1 is provided with a medical trolley 3 and is thus configured to be movable. The remote control apparatus 2 is provided at a location away from the medical manipulator 1. The medical manipulator 1 is configured to be remotely operated by the remote control apparatus 2. An operator (such as a doctor) inputs to the remote control apparatus 2 an instruction that causes the medical manipulator 1 to perform a desired operation. The remote control apparatus 2 transmits the input instruction to the medical manipulator 1. The medical manipulator 1 operates in response to the received instruction. The medical manipulator 1 is disposed in a surgery room, as a sterile field, which is sterilized.

The remote control apparatus 2 is disposed inside the surgery room or outside the surgery room, for example. The remote control apparatus 2 includes operation handles 21, foot pedals 22, a touch panel 23, a monitor 24, a support arm 25, and an armrest 26. The operation handles 21 are hand controllers (HC) or handles provided for the operator (such as a doctor) to input instructions.

The operation handles 21 are configured to operate the medical instruments 4. Specifically, the operation handles 21 receive an amount of movement input by the operator O to operate the medical instruments 4. The operation handles 21 include an operation handle 21L, which is arranged on the left side of the operator(such as a doctor) and is to be operated by the left hand of the operator O, and an operation handle 21R, which is arranged on the right side of the operator and is to be operated by the right hand of the operator O.

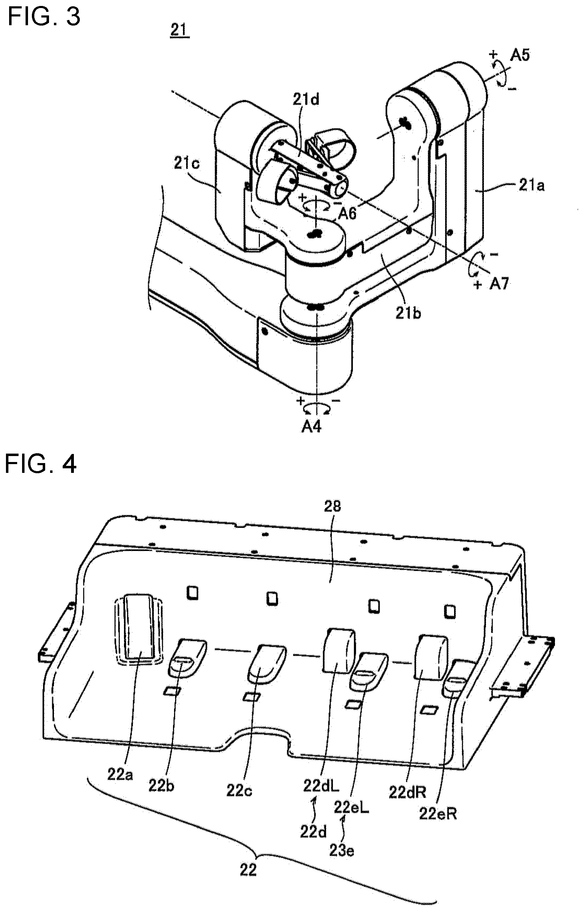

As illustrated in FIG. 3, each of the operation handles 21 includes a link portion 21a, a link portion 21b, a link portion 21c, and a link portion 21d that is to be operated by the operator (such as a doctor or the like). The link portion 21a is rotatable about an axis (joint) A4. By rotating the link portion 21a around the axis A4, the arm portion 61 described later rotates about an axis (joint) JT4. The link portion 21b is rotatable about an axis (joint) A5 with respect to the link portion 21a. By rotating the link portion 21b around the axis A5, the arm portion 61 described later rotates about an axis (joint) JT5. The link portion 21c is rotatable about an axis (joint) A6 with respect to the link portion 21b. By rotating the link portion 21c around the axis A6, the arm portion 61 rotates about an axis (joint) JT6. The link portion 21d is rotatable about an axis (joint) A7 with respect to the link portion 21c. By rotating the link portion 21d around the axis A7, the arm portion 61 rotates about an axis (joint) JT7.

Further, a movement amount of the robot arm 60 (medical instrument 4) is scaled (changed) with respect to the operation amount received by the operation handle 21. For example, when the movement scaling ratio is set to 1/2, the medical instrument 4 moves 1/2 of the movement distance of the operation handle 21. This allows for precise fine surgery.

As illustrated in FIG. 4, the plural foot pedals 22 are provided to execute functions of the medical instrument 4. The plural foot pedals 22 are arranged on a base 28. The foot pedals 22 include a switch pedal 22a, a clutch pedal 22b, a camera pedal 22c, cutting pedals 22d, and coagulation pedals 22e. The switch pedal 22a, the clutch pedal 22b, the camera pedal 22c, the cutting pedals 22d, and the coagulation pedals 22e are operated by the foot of the operator. The cutting pedals 22d includes a cutting pedal 22dR for the right robot arm 60 and a cutting pedal 22dL for the left robot arm 60. The coagulation pedals 22e include a coagulation pedal 22eR for the right robot arm 60 and a coagulation pedal 22eL for the left robot arm 60.

The switch pedal 22a is configured to select one of the robot arms 60 that is to be operated by the operation handles 21. The clutch pedal 22b is configured to perform a clutch operation that temporarily disconnects the operational connection between the robot arm 60 and the operation handle 21. While the clutch pedal 22b is depressed by the operator, the operation by the operation handle 21 is not transmitted to the robot arm 60.

The camera pedal 22c is provided for inputting a command that allows the endoscope 6 to be moved. Specifically, in response to the camera pedal 22c being depressed (stepped) by the operator, the command that allows the endoscope 6 to be moved is inputted. That is, while the command that enables the endoscope 6 to move is being inputted by the camera pedal 22c (that is, while the camera pedal 22c is depressed by the operator), the endoscope 6 is able to be moved by moving both of the operation handle 21R and operation handle 21L.

While the cutting pedal 22d (coagulation pedal 22e) is depressed (stepped) by the operator, an electrosurgical device (not illustrated) is activated.

As illustrated in FIG. 1, the monitor 24 is a display device of a scope type configured to display an image captured by the endoscope 6. The support arm 25 supports the monitor 24 in such a manner that the height of the monitor 24 is adjusted to the height of the face of the operator (such as a doctor). The touch panel 23 is disposed on the armrest 26. When a sensor(s) provided in the vicinity of the monitor 24 detects the head of the operator, the medical manipulator 1 is allowed to be operated by the remote control apparatus 2. The operator operates the operation handles 21 and the foot pedals 22, while viewing the surgical site (or affected area) displayed on the monitor 24. With this, the instruction is input to the remote control apparatus 2. The instruction that is input to the remote control apparatus 2 is transmitted to the medical manipulator 1

The medical trolley 3 is provided with a control unit 31 (circuitry and/or processor) that controls the operation of the medical manipulator 1 and a storage 32 that stores therein programs for controlling the operation of the medical manipulator 1. Based on the instruction inputted to the remote control apparatus 2, the control unit 31 of the medical trolley 3 controls the operation of the medical manipulator 1.

Further, the medical trolley 3 is provided with an input device 33. The input device 33 is configured to accept operations to move or change posture of a positioner 40, an arm base 50, and robot arms 60, mainly to prepare for surgery before the surgery.

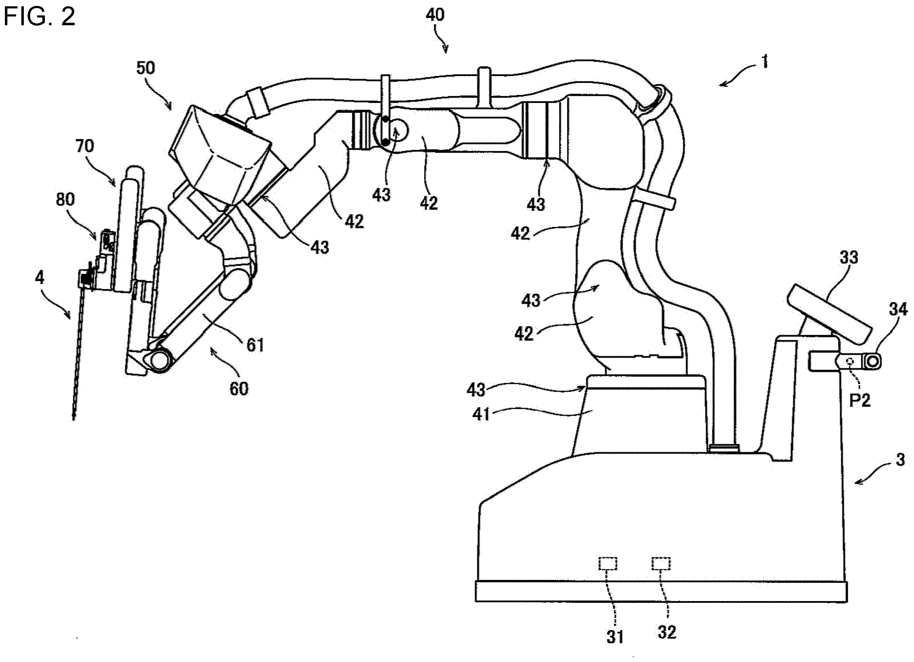

As illustrated in FIGS. 1 and 2, the medical manipulator 1 is disposed in the surgery room. The medical manipulator 1 includes the medical trolley 3, the positioner 40, the arm base 50, and the robot arms 60. The arm base 50 is attached to a distal end of the positioner 40. The arm base 50 is a relatively long rod shape (elongate shape). Base portions (proximal end portions) of the robot arms 60 are attached to the arm base 50. Each of the robot arms 60 is configured such that the robot arm 60 is able to take a folded posture (storage posture). The arm base 50 and the robot arms 60 are used with being covered with a sterile drape (not illustrated). The robot arm 60 supports the medical instrument 4.

The positioner 40 is configured as a 7-axis articulated robot. The positioner 40 is disposed on the medical trolley 3. The positioner 40 is configured to move the arm base 50. Specifically, the positioner 40 is configured to move the position of the arm base 50 three-dimensionally.

The positioner 40 includes a base portion 41 and link portions 42 connected to the base portion 41. The link portions 42 are connected to each other via joints 43.

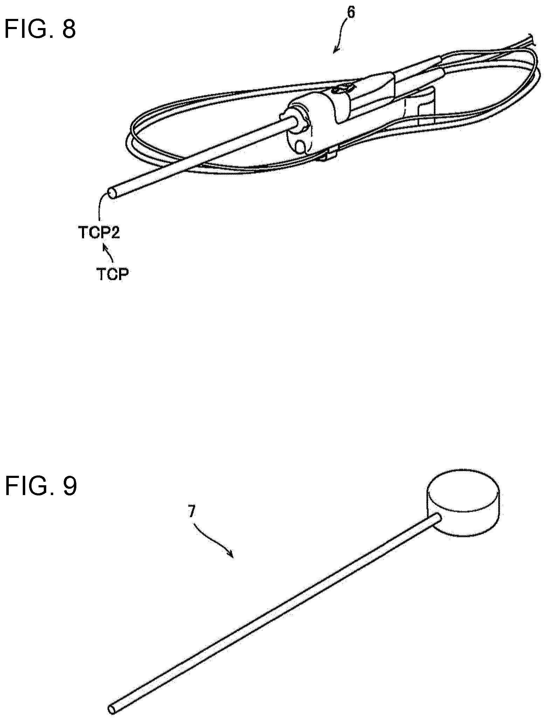

As illustrated in FIG. 1, to the distal end of each of the robot arms 60, the medical instrument 4 is attached. The medical instruments 4 include, for example, instruments that are replaceable, an endoscope 6 (see FIG. 8) configured to image a surgical site (that is, to capture an image of a surgical site), and the like.

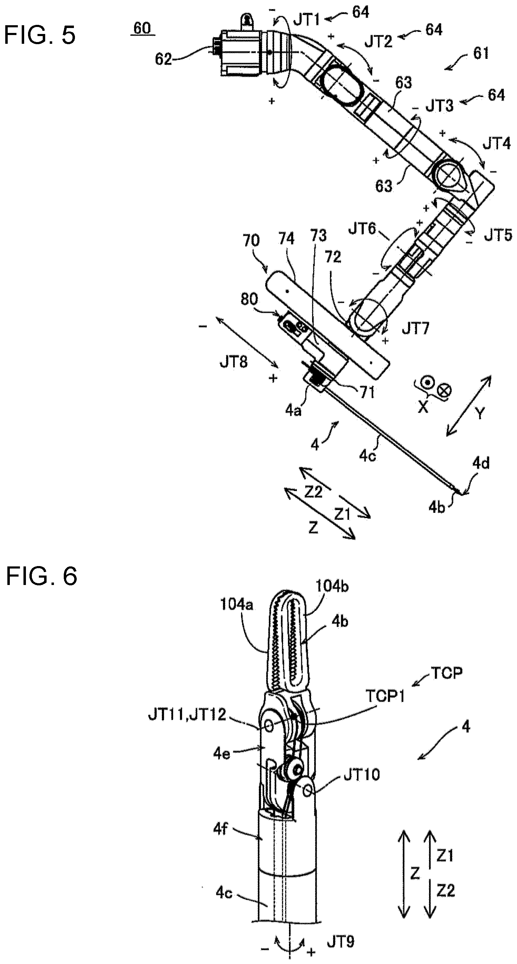

As illustrated in FIG. 5, the instrument is provided with a driven unit 4a, which is driven by servomotors M2 provided in a holder 71 of the robot arm 60. To the distal end of the surgical instrument, an end effector 4b is provided.

As illustrated in FIG. 6, the instrument includes: a first support 4e having a distal end portion thereof that rotatably supports proximal end portions of jaw members 104a and 104b about an axis (joint) JT11; a second support 4f having a distal end portion thereof that rotatably supports a proximal end portion of the first support 4e about an axis (joint) JT10; and a shaft 4c connected a proximal end portion of the second support 4f. The driven unit 4a, the shaft 4c, the second support 4f, the first support 4e, and the end effector 4b are arranged along the Z direction. The axis JT11 is orthogonal to a direction (Z direction) in which the shaft 4c extends. The axis JT10 is provided away from the axis JT11 in the direction in which the shaft 4c extends, and is orthogonal to the axis JT11 and orthogonal to the direction in which the shaft 4c extends.

The end effector 4b is attached to the first support 4e so as to be rotatable about the axis JT11. The second support 4f rotatably supports the first support 4e about the axis JT10. In other words, the first support 4e is attached to the second support 4f so as to be rotatable about the axis JT10. The first support 4e is a clevis whose distal side (Z1 side) portion has a U-shape. A tool center point (TCP1) is set at the center of the U-shaped distal side portion of the first support 4e along the axis JT11. The second support 4f is a clevis whose distal side (Z1 side) portion has a U-shape.

The medical instrument 4 includes an axis (joint) JT9 as a rotation axis of the shaft 4c (extending along the direction in which the shaft 4c extends) and an axis (joint) JT12 about which the end effector 4b open and close. Note that the plural (for example, four) servomotors M2 are provided in the holder 71 of the robot arm 60 and rotors (rotation members) in the driven unit 4a are driven by the plural servomotors M2. As a result, the medical instrument 4 is driven about the J9 axis to the J12 axis.

As illustrated in FIG. 8, a tool center point TCP2 of the endoscope 6 is set at the distal end of the endoscope 6.

Next, a configuration of the robot arm 60 is described in detail.

As illustrated in FIG. 5, the robot arm 60 includes an arm portion 61 (a base portion 62, link portions 63, joint portions 64) and a translation movement mechanism 70 provided at the distal end portion of the arm portion 61. The robot arm 60 is configured such that the distal end side thereof is three-dimensionally movable with respect to the proximal side (the arm base 50) thereof. The arm portion 61 is configured as a 7-axis articulated robot arm. The plural robot arms 60 have the same configuration as each other.

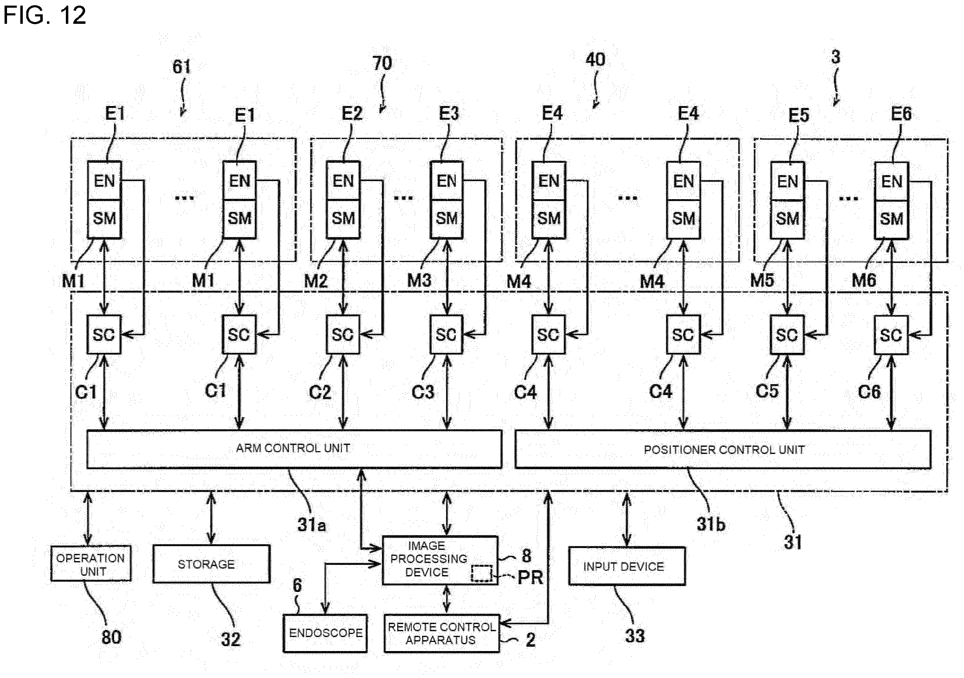

As illustrated in FIG. 5, the robot arm 60 includes the axis (joints) JT1 to JT7 as rotation axes and an axis (joint) J8 as a linear motion axis. The axes JT1 to JT7 correspond to the rotation axes of the joint portions 64 of the arm portion 61. The axis JT7 corresponds to the proximal end side link portion 72 of the translational movement mechanism 70. An axis (joint) JT8 is an axis for moving the distal end side link portion 73 of the translational movement mechanism 70 relative to the proximal end side link portion 72 along the Z direction. That is, the servomotors M1 illustrated in FIG. 12 are provided to correspond to the joints JT1 to JT7 of the robot arm 60. The servomotor M3 is provided to correspond to the joint JT8.

The translation movement mechanism 70 is provided on a side of the distal end of the arm portion 61. The medical instrument 4 is attached to the translation movement mechanism 70. The translation movement mechanism 70 translationally moves the medical instrument 4 in the insertion direction of the medical instrument 4 into a patient P. The translation movement mechanism 70 is configured to translationally move the medical instrument 4 relative to the arm portion 61. Specifically, the translation movement mechanism 70 is provided with the holder 71 configured to hold the medical instrument 4. The holder 71 accommodates therein the servo- motors M2 (see FIG. 12).

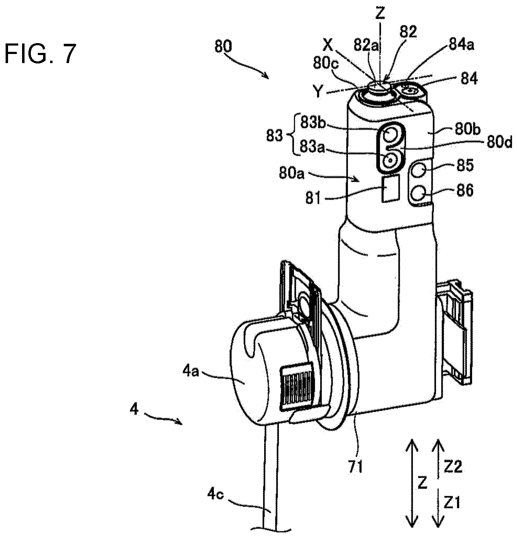

As illustrated in FIG. 7, the medical manipulator 1 includes an operation unit 80 which is attached to each of the robot arms 60 to operate the robot arm 60. The operation unit 80 includes an enable switch 81, a joystick 82, and a switch section 83. The enable switch 81 enables or disables the movements of the robot arm 60 in response to the joystick 82 and the switch section 83. When the enable switch 81 is depressed by an operator (nurse, assistant, etc.) gripping the operation unit 80, the enable switch 81 enables the medical instrument 4 to move by the robot arm 60.

The switch section 83 includes: a switch 83a for moving the medical instrument 4 in the direction in which the medical instrument 4 is inserted into the patient P along the longitudinal direction of the medical instrument 4; and a switch 83b for moving the distal end 4d of the medical instrument 4 in the direction opposite to the direction in which the medical instrument 4 is inserted into the patient P. Both the switch 83a and the switch 83b are composed of push button switches.

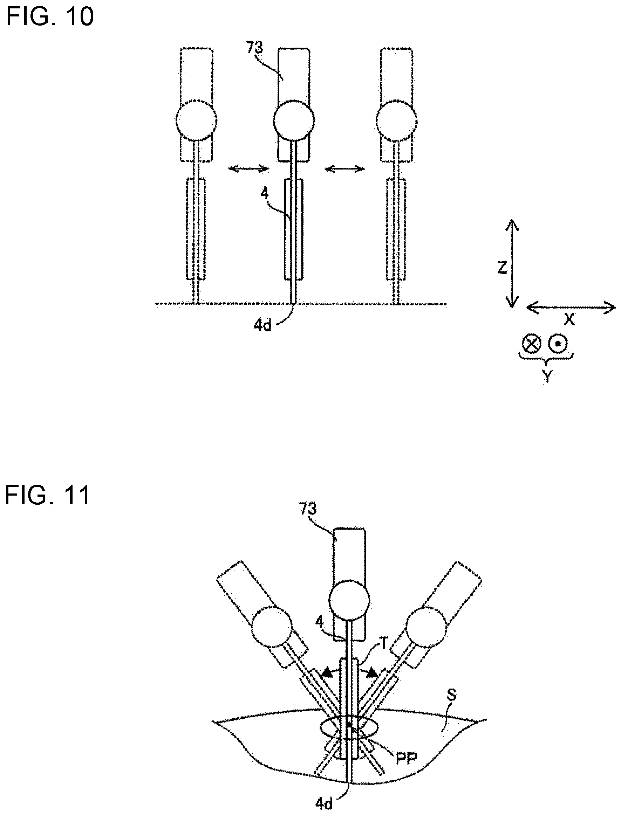

As illustrated in FIG. 7, the operation unit 80 includes a pivot button 85 for setting a pivot position PP that serves as a fulcrum (see FIG. 11) for the movement of the medical instrument 4 attached to the robot arm 60. The pivot button 85 is provided on a surface 80b of the operation unit 80 so as to be adjacent to the enable switch 81. The pivot position PP is set by pressing the pivot button 85 in a state where the distal end of the endoscope 6 (see FIG. 8) or the distal end of the pivot position setting device 7 (FIG. 9) is moved to a position corresponding to an insertion position of the trocar T inserted into the body surface S of the patient P. The pivot position PP set is stored in the storage 32. In the teaching of the pivot position PP, the pivot position PP is set as one point (coordinate), but the teaching of the pivot position PP does not set the direction of the medical instrument 4.

As illustrated in FIG. 1, the endoscope 6 is attached to one (for example, the robot arm 60c) of the plural robot arms 60, and the medical instruments 4 other than the endoscope 6 are attached to the other robot arms 60 (for example, the robot arms 60a, 60b, and 60d). Specifically, for surgery, the endoscope 6 is attached to one of the four arms 60, and the medical instruments 4 other than the endoscope 6 are attached to the other three arms 60. In the state where the endoscope 6 is attached to the robot arm 60, the pivot position PP for the endoscope 6 is set to the robot arm 60 to which the endoscope 6 is attached. Further, in the state where the pivot position setting device 7 is attached to the robot arm 60 to which the medical instrument 4 other than the endoscope 6 is attached, the pivot position PP for the medical instrument 4 is set to the robot arm 60 to which the medical instrument 4 other than the endoscope 6 is attached. The endoscope 6 is attached to one of two robot arms 60 (robot arms 60b and 60c) arranged in a central area among the four robot arms 60 arranged adjacent to each other. That is, the pivot position PP is individually set for each of the plurality of robot arms 60.

As illustrated in FIG. 7, the surface 80b of the operation unit 80 is provided with an adjustment button 86 for optimizing the position of the robot arm 60. After the pivot position PP is set to the robot arm 60 to which the endoscope 6 is attached, the positions of the other robot arms 60 (arm bases 50) are optimized by pressing the adjustment button 86.

As illustrated in FIG. 7, the operation unit 80 includes a mode switch button 84 for switching between a translational movement mode (see FIG. 10) to translationally move the medical instrument 4 attached to the robot arm 60 and a rotational movement mode (see FIG. 11) for rotationally move the medical instrument 4 attached to the robot arm 60. In the vicinity of the mode switch button 84, a mode indicator 84a is provided. The mode indicator 84a indicates the switched mode between the translational movement mode and the rotational movement mode. Specifically, when the mode indicator 84a is turned on (rotational movement mode) or turned off (translational movement mode), the current mode (translational movement mode or rotational movement mode) is indicated.

Further, the mode indicator 84a also serves as a pivot position indicator that indicates that the pivot position PP has been set.

As illustrated in FIG. 10, in the translational movement mode to translationally move the robot arm 60, the robot arm 60 is moved in such a manner that the distal end 4d of the medical instrument 4 moves on the XZ plane. Further, as illustrated in FIG. 11, in the rotational movement mode in which the medical instrument 4 is to be rotationally moved, when the pivot position PP is not set by the operator, the robot arm 60 is moved such that the medical instrument 4 is rotated around the end effector 4b, and when the pivot position PP is set by the operator, the robot arm 60 is moved such that the medical instrument 4 is rotated around the pivot position PP as a fulcrum. The medical instrument 4 is rotationally moved in the state where the shaft 4c of the medical instrument 4 is inserted in the trocar T.

As illustrated in FIG. 12, the robot arm 60 is provided with the plurality of servomotors M1, a plurality of encoders E1, and a plurality of speed reducers (not illustrated), so as to correspond to the plurality of joint portions 64 of the arm portion 61. The encoder E1 is configured to detect the rotation angle of the servomotor M1. The speed reducer is configured to reduce the rotation of the servomotor M1 to increase the torque.

As illustrated in FIG. 12, the translational movement mechanism 70 includes the servomotors M2 for rotating the rotors (rotation members) provided in the driven unit 4a of the medical instrument 4, a servomotor M3 for translationally moving the medical instrument 4, encoders E2, an encoder E3, and speed reducers (not illustrated). The encoders E2 and the encoder E3 are configured to detect the rotation angles of the servomotors M2 and the servomotor M3, respectively. The speed reducers are configured to reduce the rotations of the servomotors M2 and the servomotor M3 to increase the torque thereof.

The positioner 40 is provided with a plurality of servomotors M4, a plurality of encoders E4, and a plurality of speed reducers (not illustrated), so as to correspond to the plurality of joints 43 of the positioner 40. The encoders E4 detect the rotation angles of the servomotors M4. The speed reducers are configured to reduce the rotations of the servomotors M4 to increase the torque thereof.

The medical trolley 3 is provided with servomotors M5 that drive a plurality of front wheels (not illustrated) of the medical trolley 3 respectively, encoders E5, speed reducers (not illustrated), and brakes (not illustrated). The speed reducer is configured to reduce the rotation of the servomotor M5 to increase the torque. A throttle 34a of the medical trolley 3 is provided with a potentiometer P1 (see FIG. 1). The servomotors M5 for the front wheels are driven based on the rotation angle detected by the potentiometer P1 according to the rotation of the throttle 34a. The rear wheels (not illustrated) of the medical trolley 3 are a twin-wheel type and are steered based on the left-right rotation of an operation handle 34. The operation handle 34 of the medical trolley 3 is provided wit a potentiometer P2 (see FIG. 2). The rear wheels of the medical trolley 3 are provided with servomotors M6, encoders E6, and speed reducers (not illustrated). The speed reducer is configured to reduce the rotation of the servomotor M6 to increase the torque. The servomotors M6 for the rear wheels are driven based on the rotation angle detected by the potentiometer P2 according to the left-right rotation of the operation handle 34. That is, the steering of the rear wheels by the left-right rotation of the operation handle 34 is power-assisted by the servomotors M6.

Further, the medical trolley 3 moves in the front-rear direction by driving the front wheels. By rotating the operation handle 34 of the medical trolley 3, the rear wheels of the medical trolley 3 are steered and thus the medical trolley 3 is rotated in the left-right direction.

The control unit 31 of the medical trolley 3 includes an arm control unit 31a that controls the movements of the plurality of robot arms 60 based on commands, and a positioner control unit 31b that controls the movement of the positioner 40 and driving of the front wheel (not illustrated) of the medical trolley 3 based on commands. A servo control unit C1 that controls the servomotors M1 for driving the robot arm 60 is electrically connected to the arm control unit 31a. Further, an encoder E1 that detects the rotation angle of the servomotor M1 is electrically connected to the servo control unit C1.

A servo control unit C2 that controls the servomotors M2 for driving the medical instrument 4 is electrically connected to the arm control unit 31a. The encoders E2 that detect the rotation angles of the servomotors M2 are electrically connected to the servo control unit C2. The servo control unit C3 that controls the servomotor M3 for translationally moving by the translational movement mechanism 70 is electrically connected to the arm control unit 31a. The encoder E3 for detecting the rotation angle of the servomotor M3 is electrically connected to the servo control unit C3.

The operation command input to the remote control apparatus 2 is input to the arm control unit 31a. The arm control unit 31a generates position commands based on the operation command inputted and the rotation angles detected by the encoders E1 (E2, E3), and outputs the position commands to the servo control units C1 (C2, C3). The servo control units C1 (C2, C3) generate torque commands based on the position commands inputted from the arm control unit 31a and the rotation angles detected by the encoders E1 (E2, E3), and output the torque commands to the servomotors M1 (M2, M3). As a result, the robot arm 60 is moved so as to comply with the operation command inputted to the remote control apparatus 2.

As illustrated in FIG. 12, the control unit 31 (arm control unit 31a) is configured to operate the robot arm 60 based on an input signal from the joystick 82 of the operation unit 80. Specifically, the arm control unit 31a generates position commands based on the input signal (operation command) input from the joystick 82 and the rotation angles detected by the encoders E1, and outputs the position commands to the servo control units C1. The servo control unit C1 generates torque commands based on the position command input from the arm control unit 31a and the rotation angles detected by the encoders E1, and outputs the torque commands to the servomotors M1. As a result, the robot arm 60 is moved so as to follow the operation command input to the joystick 82.

The control unit 31 (arm control unit 31a) is configured to operate the robot arm 60 based on an input signal from the switch section 83 of the operation unit 80. Specifically, the arm control unit 31a generates position commands based on the input signal (operation command) input from the switch section 83 and the rotation angles detected by the encoders E1 or E3, and outputs the position commands to the servo control units C1 or C3. The servo control units C1 or C3 generate torque commands based on the position command input from the arm control unit 31a and the rotation angles detected by the encoders E1 or E3, and outputs the generated torque commands to the servomotors M1 or M3. As a result, the robot arm 60 is moved so as to follow the operation command inputted to the switch section 83.

As illustrated in FIG. 12, the servo control units C4 that control the servomotors M4 for moving the positioner 40 is electrically connected to the positioner control unit 31b. The encoders E4 that detects the rotation angles of the servomotors M4 are electrically connected to the servo control units C4. The servo control units C5 that control the servomotors 5 for driving the front wheel (not illustrated) of the medical trolley 3 are electrically connected to the positioner control unit 31b. The encoders E5 that detects the rotation angles of the servomotors M5 are electrically connected to the servo control units C5.

An operation command regarding setting of the preparation position and the like is input from the input device 33 to the positioner control unit 31b. The positioner control unit 31b generates position commands based on the operation command inputted from the input device 33 and the rotation angle detected by the encoder E4, and outputs the position commands to the servo control units C4. The servo control unit C4 generates torque commands based on the position command input from the positioner control unit 31b and the rotation angles detected by the encoders E4, and outputs the torque commands to the servomotors M4. As a result, the positioner 40 is moved so as to follow the operation command input to the input device 33. Similarly, the positioner control unit 31b moves the medical trolley 3 based on the operation command from the input device 33.

The surgical operation system 100 includes an image processing device 8. The image processing device 8 is configured to obtain the image captured by the endoscope 6 and displays the captured image obtained from the endoscope 6 on the monitor 24 of the remote control apparatus 2.

### (Configurations of Medical equipment, Adaptor, Drape, and Arm)

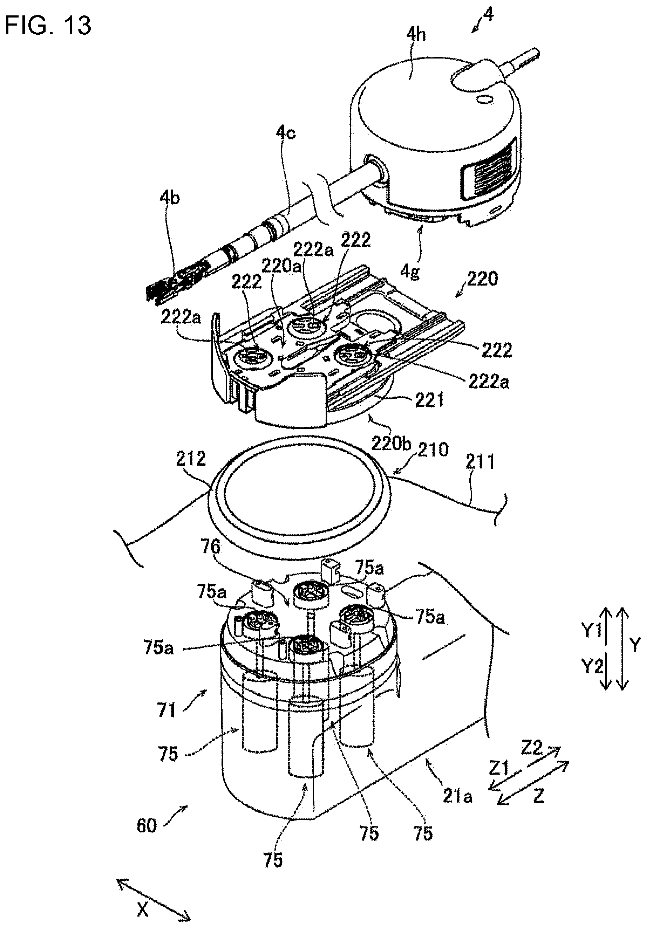

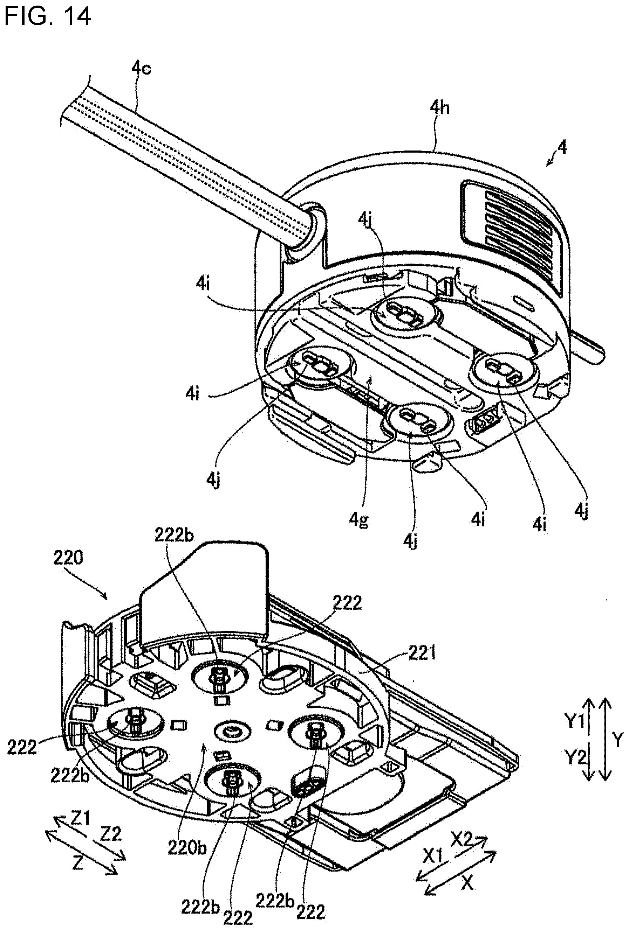

With reference to FIGS. 13 and 14, the configurations of the medical instrument 4, an adaptor 220, a drape 210, and the robot arm 60 are described.

As illustrated in FIGS. 13 and 14, the medical instrument 4 is detachably connected to the robot arm 60 through the adaptor 220. The adaptor 220 is arranged between the holder 71 (driving parts 75) of the robot arm 60 and the medical instrument 4. The adaptor 220 is a drape adaptor for holding the drape 210 and is to be replaced by the user after each surgery. Accordingly, the drape 210 can be held by using the adaptor 220. The drape 210 is for covering the robot arm 60 and is sterilized. The adaptor 220 is configured to put the drape 210 between the adaptor 220 and the robot arm 60.

The medical instrument 4 includes a connection portion 4g, serving as an attachment surface, provided on the Y2 side of the driven unit 4a of the medical instrument 4, and the connection portion 4g of the medical instrument 4 is to be attached to and connected to the adaptor 220. The connection portion 4g is provided at a housing 4h of the driven unit 4a and is attached to the robot arm 60 via the adaptor 220. The adaptor 220 includes a connection portion 220a, serving as an attachment surface, provided on the Y1 side of the adaptor 220, and the medical instrument 4 is to be attached to and connected to the connection portion 220a of the adaptor 220. The adaptor 220 further includes a connection portion 220b, serving as an attachment surface, provided on the Y2 side of the adaptor 220, and the connection portion 220b of the adaptor 220 is attached and connected to the holder 71 (driving parts 75) of the robot arm 60. The holder 71 (driving parts 75) of the robot arm 60 includes a connection portion 76, serving as an attachment surface, provided on the Y1 side of the robot arm 60, and the adaptor 220 is attached and connected to the connection portion 76 of the robot arm 60.

As illustrated in FIG. 13, the robot arm 60 is used in a clean area and is thus covered with the drape 210. In operation rooms, clean technique is used in order to prevent surgical incision sites and the medical equipment from being contaminated by pathogen, foreign matters, or the like. The clean technique defines a clean area and a contaminated area, which is outside the clean area. The surgery sites are located in the clean area. Members of the surgical team including the operator make sure that only sterile objects are placed in the clean area during surgery and perform sterilization for an object which is to be moved to the clean area from the contaminated area. Similarly, when the members of the surgical team including the operator place their hands in the contaminated area, the members sterilize their hands before directly touching objects located in the clean area. Instruments used in the clean area are sterilized or are covered with sterile drapes 210.

The drape 210 includes a body section 211 that covers the robot arm 60 and an attachment section 212 that is sandwiched between the driving parts 75 of the robot arm 60 and the adaptor 220. The body section 211 is made of a flexible film member. The flexible film member is made of a resin material, such as thermoplastic polyurethane and polyethylene. The body section 211 includes an opening such that the driving parts 75 of the robot arm 60 are engageable with the adaptor 220. To the opening of the body section 211, the attachment section 212 is provided so as to close the opening. The attachment section 212 is made of a resin mold member. The resin mold member is made of a resin member such as polyethylene terephthalate. The attachment section 212 is harder (less flexible) than the body section 211. The attachment section 212 includes an opening such that the driving parts 75 of the robot arm 60 are engageable with the adaptor 220. The opening of the attachment section 212 may be provided corresponding to a portion where the driving parts 75 of the robot arm 60 are engaged with the adaptor 220. The opening of the attachment section 212 may include plural openings corresponding to plural portions at which the driving parts 75 of the robot arm 60 are engaged with the adaptor 220.

As illustrated in FIGS. 13 and 14, the adaptor 220 includes an adaptor main body 221 and plural (four) drive transmission members 222 supported by the adaptor main body 221 to be rotatable about respective rotational axes extending in the Y direction with respect to the adaptor main body 221. The plural drive transmission members 222 are provided in the adaptor main body 221 to be rotatable about their rotation axes. The number (four) of the plural drive transmission members 222 provided corresponds to the number (four) of plural driven members 4i of the medical instrument 4. The drive transmission members 222 are configured to transmit driving forces from the robot arm 60 to the driven members 4i of the medical instrument 4. Each of the drive transmission members 222 include a fitting recess 222a, which is to be fitted with a fitting protrusion 4j of the corresponding driven member 4i of the medical instrument 4. The fitting recess 222a is provided at a surface of the drive transmission member 222 on the Y1 side (the medical instrument 4 side) and is recessed from the Y1-side surface of the drive transmission member 222 toward a side (the Y2 side) opposite to the medical instrument 4 side.

Each of the drive transmission members 222 include a fitting recess 222b, which is to be fitted with a fitting protrusion 75a of the corresponding driving parts 75 of the robot arm 60. The fitting recess 222b is provided at a surface of the drive transmission member 222 on the Y2 side (the robot arm 60 side) and is recessed from the Y2-side surface of the drive transmission member 222 toward a side (the Y1 side) opposite to the robot arm 60 side.

### (Configuration of Manual Surgical Instrument)

A configuration of a manual surgical instrument 200 is described below. The manual surgical instrument 200 is originally an instrument manually operated by an operator such as a doctor. However, in a first embodiment, the manual surgical instrument 200 is not only directly operated by the operator, but also is operated via the medical manipulator 1 by the remote control apparatus 2.

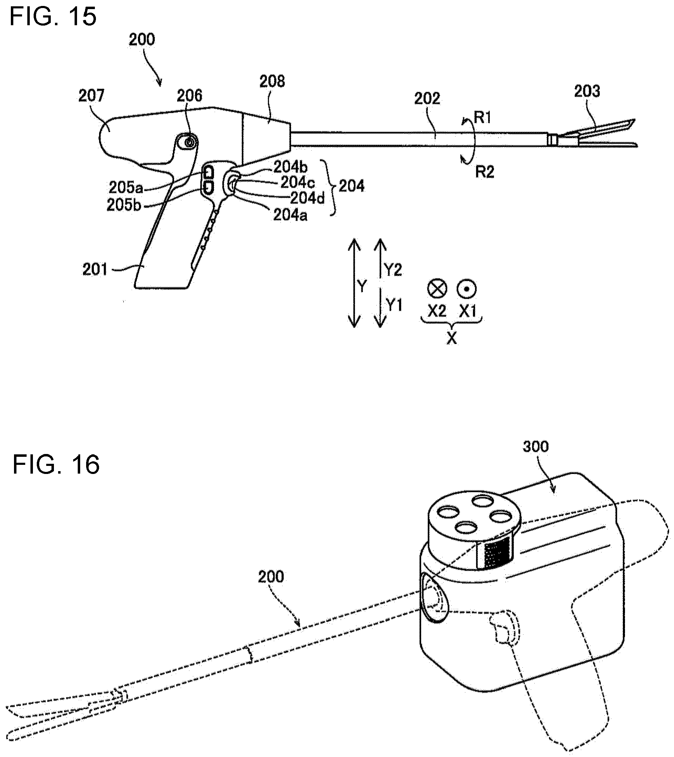

With reference to FIG. 15, the manual surgical instrument 200 to be attached to the robot arm 60 is described below. The manual surgical instrument 200 can be attached to the robot arm 60 in place of the dedicated medical instrument 4. The manual surgical instrument 200 is, for example, a battery-powered stapler instrument configured to be driven by a battery. Note that the manual surgical instrument 200 may be a manual surgical instrument other than the stapler instrument. The manual surgical instrument 200 includes a grip portion 201, a shaft 202, and an end effector 203 that is provided at the distal end of the shaft 202. The end effector 203 includes a pair of jaw members consisting of a reload housing and an anvil that opens and closes with respect to the reload housing. Note that the manual surgical instrument 200 may be driven by a lever or the like operated by a finger(s) of the operator such as a doctor or the like.

A first switch 204 (a cross key) is provided on a front surface of the grip portion 201 (the surface on the shaft 202 side). The first switch 204 is a cross button including a switch portion 204a provided on the Y1 side, a switch portion 204b provided on the Y2 side, a switch portion 204c provided on the X1 side, and a switch portion 204d provided on the X2. The switch portions 204a, 204b, 204c, and 204d are pressed in the Z2 direction by first pressing portion 331 and second pressing portion 332, which will be described later. Further, the first switch 204 is an example of an "operation portion" or a "cross-shaped operation portion."

A pair of second switches 205a and 205b and a third switch 206 are provided on a side surface of the grip portion 201. When a Y1 side portion (the switch portion 204a) of the first switch 204 is pressed by the operator such as a doctor or the like, the jaws of the end effector 203 are closed. When a Y2 side portion (the switch portion 204b) of the first switch 204 is pressed by the operator, the jaws of the end effector 203 are opened. When an X1 side portion (the switch portion 204c) of the first switch 204 is pressed by the operator, the end effector 203 is bent (swung) toward the X1 side with respect to the shaft 202. When an X2 side portion (the switch portion 204d) of the first switch 204 is pressed by the operator, the end effector 203 is bent (swung) toward the X2 side with respect to the shaft 202.

When the second switch 205a is pressed by the operator, the shaft 202 rotates clockwise (in the R1 direction), whereby the end effector 203 rotates clockwise (in the R1 direction). When the second switch 205b is pressed by the operator, the shaft 202 rotates counterclockwise (in the R2 direction), whereby the end effector 203 rotates counterclockwise (in the R2 direction).

When the third switch 206 is pressed by the operator, the mode is switched to a suturing mode to suture the skin of the patient P. After that, by continuously pressing the switch portion 204a or the switch portion 204b of the first switch 204, the operation by the stapler of stitching the tissue clamped by the jaws and the operation of cutting the vicinity of the sewn portion are performed. Note that the second switches 205a and 205b and the third switch 206 are examples of "operation portions."

### (Configuration of Surgical Instrument Adaptor)

Next, a configuration of a surgical instrument adaptor 300 is described. As illustrated in FIG. 16, in a state where the manual surgical instrument 200 is attached to the surgical instrument adaptor 300, the surgical instrument adaptor 300 connects the manual surgical instrument 200 to the robot arm 60 such that the manual surgical instrument 200 is operable by the robot arm 60.

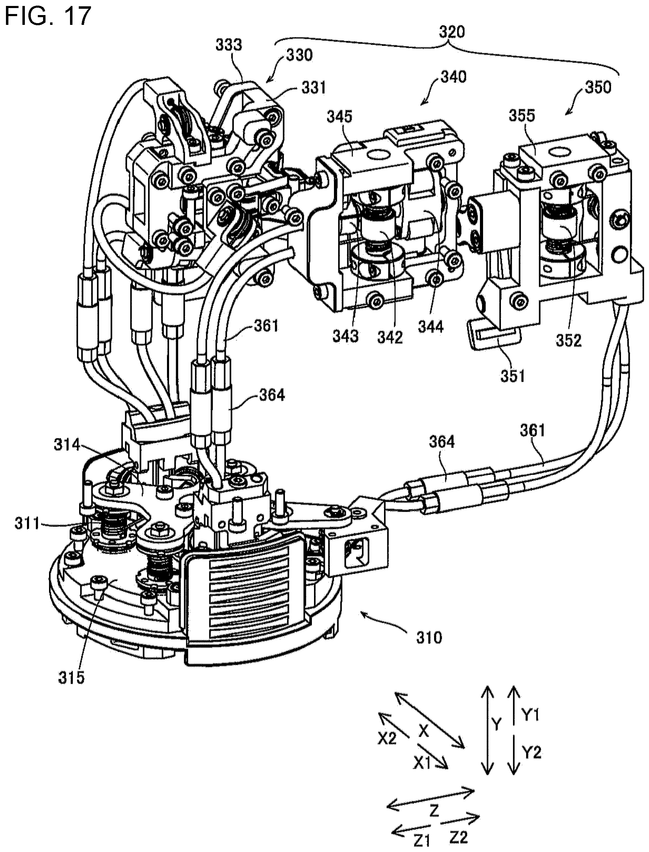

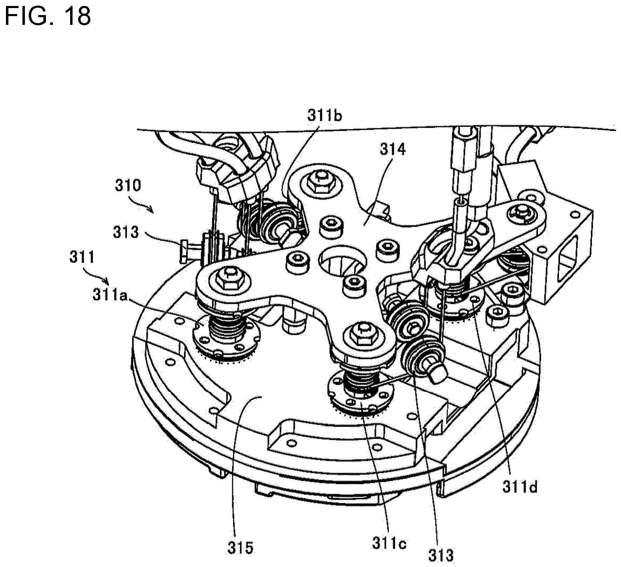

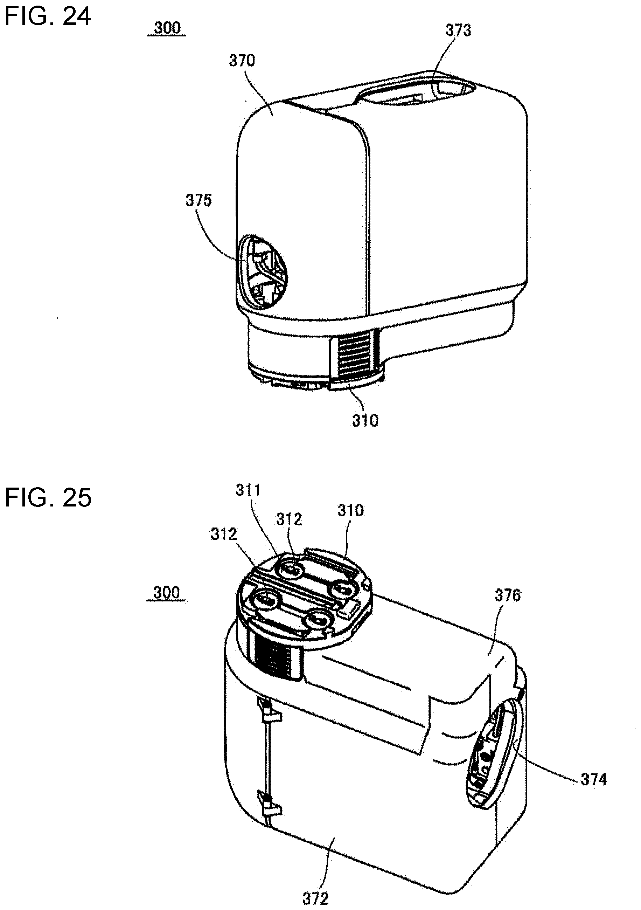

In a first embodiment, as illustrated in FIGS. 17 and 18, the surgical instrument adaptor 300 includes an interface portion 310. The interface portion 310 includes rotation members 311 to be driven to rotate by driving forces transmitted from the driving parts 75 (see FIG. 13) provided to the robot arm 60. The interface portion 310 is attached to the holder 71 (see FIG. 13) of the translational movement mechanism 70 of the robot arm 60 via the adaptor 220. Each of the rotation members 311 is composed of a pulley (capstan) around which an elongate element 360 described later is wound. The number of the rotation members 311 provided are four. Each of the rotation members 311 includes, on a surface thereof on the translational movement mechanism 70 side, a fitting projection 312 (see FIG. 25) that fits with the fitting recess 222a (see FIG. 13) of the corresponding drive transmission member 222 of the adaptor 220.

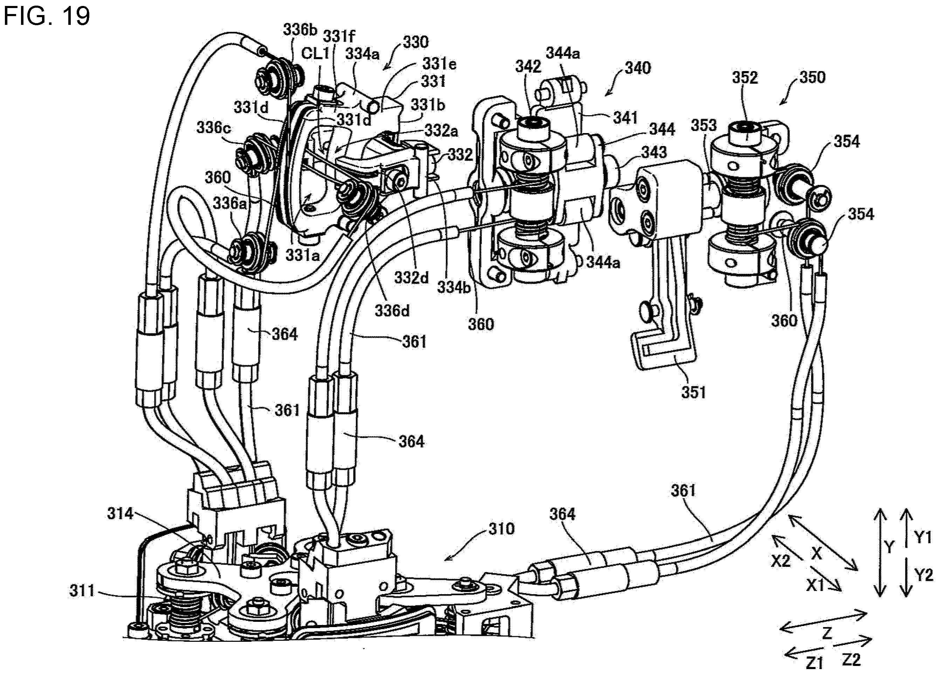

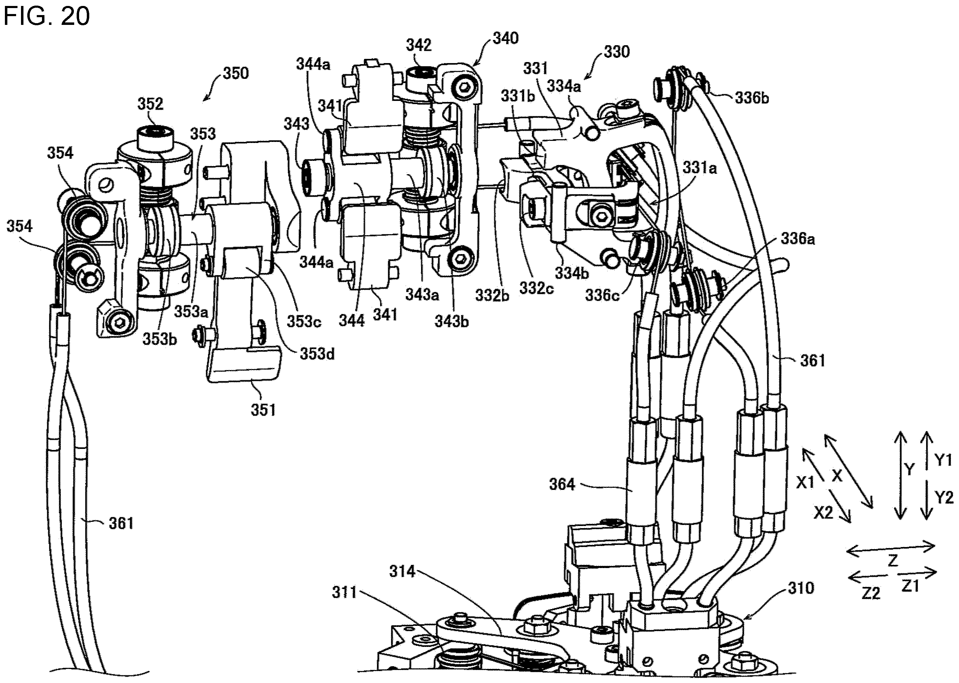

In a first embodiment, the surgical instrument adaptor 300 includes elongate elements 360 (see FIGS. 19 and 20) each of which is connected to the corresponding rotation member 311 and a driven unit 320 and is configured to transmit the driving force from the corresponding rotation member 311 to the driven unit 320. The elongate element 360 is composed of a wire, a cable, or the like. In a first embodiment, the surgical instrument adaptor 300 includes guide tubes 361 each of which guides the corresponding elongate element 360.

In a first embodiment, the surgical instrument adaptor 300 includes the driven unit 320. The driven unit 320 include pressing portions (a first pressing portion 331, a second pressing portion 332, an arm portion 341, and an arm portion 351 described later) that are configured to press down the first switch 204, the second switches 205a and 205b, and the third switch 206 (see FIG. 15) of the manual surgical instrument 200. Specifically, the driven unit 320 includes a plurality (three) of driven parts (driven devices) corresponding to the first switch 204, the second switches 205a and 205b, and the third switch 206 of the manual surgical instrument 200, and each of the plurality of driven parts includes the pressing portion(s). Each of the plurality of driven parts of the driven unit 320 is modularized. Note that "modularization" means that components constituting each driven part of the driven unit 320 are configured as one group. The first pressing portion 331, the second pressing portion 332, the arm portion 341, and the arm portion 351 are examples of "pressing portions" of the disclosure.

Specifically, the driven unit 320 includes a first driven part 330 that presses the first switch 204 of the manual surgical instrument 200, a second driven part 340 that presses the pair of the second switches 205a and 205b of the manual surgical instrument 200, and a third driven part 350 that presses the third switch 206 of the manual surgical instrument 200. The first driven part 330, the second driven part 340, and the third driven part 350 are examples of "driven parts" of the disclosure.

### (First Driven Part 330)

In a first embodiment, as illustrated in FIG. 19, the pressing portions of the first driven part 330 includes: the first pressing portion 331 that is driven to move in an arc in the YZ plane to press the first switch 204; and the second pressing portion 332 that is driven independently of the first pressing portion 331 to move in an arc in the XZ plane to press the first switch 204. The first pressing portion 331 and the second pressing portion 332 move along the operation directions of the first switch 204 of the manual surgical instrument 200.

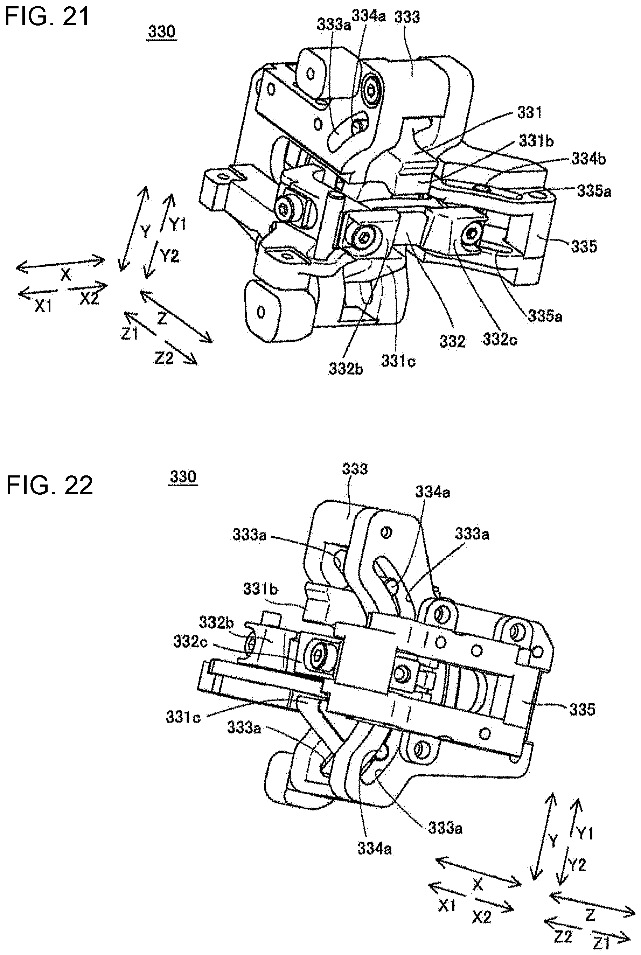

The first pressing portion 331 is arranged along the YZ plane. The first pressing portion 331 includes a first portion 331e formed in a C-shape that opens toward the Z2 side and a second portion 331f formed in a substantially annular shape having an opening 331a (a hole) at a center portion thereof. The first pressing portion 331 is provided with a first protrusion 331b and a second protrusion 331c (see FIGS. 21 and 22) that protrude toward the first switch 204. The first protrusion 331b is provided at one end of the C-shape of the first portion 331e, and is configured to press the switch portion 204a (the Y1 side) of the first switch 204. The second protrusion 331c is provided at the other end of the C-shape of the first portion 331e, and is configured to press the switch portion 204b (the Y2 side) of the first switch 204.

As illustrated in FIG. 19, the second pressing portion 332 is arranged along the XZ plane. The second pressing portion 332 has a substantially C-shape with an opening 332a that opens toward the Z1 side. The opening 332a of the C-shape of the second pressing portion 332 is arranged at the opening 331a of the first pressing portion 331. The second pressing portion 332 further includes, at a Z2 side portion thereof, a third protrusion 332b and a fourth protrusion 332c (see FIG. 21) that protrude toward the first switch 204. The third protrusion 332b of the second pressing portion 332 presses the switch portion 204c (the X1 side) of the first switch 204. The fourth protrusion 332c of the second pressing portion 332 presses the switch portion 204b (the X2 side) of the first switch 204.

Further, in a first embodiment, the first driven part 330 includes, as illustrated in FIGS. 21 and 22, a frame 333 that supports the first pressing portion 331. The frame 333 is configured such that the first pressing portion 331 is sandwiched by the frame 333 in the X direction. The frame 333 includes guide portions 333a each of which is formed as a hole extending along an arc shape. The guide portions 333a are provided at an X1 side portion and an X2 side portion of the frame 333 so as to form pairs in the X direction. That is, the pairs of guide portions 333a are provided so as to be opposed to each other in the X direction. A pair of guide portions 333a are provided in a Y1 side portion of the frame and a pair of guide portions 33a are provided in a Y2 side portion of the frame 333. That is, the number of the guide portions 333a provided are four. Each of a Y1-side end portion and a Y2 side end portion of the first pressing portion 331 include an engagement portion 334a formed in a pin shape extending in the X direction. Each engagement portions 334a are inserted in the pair of guide portions 333a and thus is engaged with the pair of guide portions 333a. The first pressing portion 331 is configured to be movable in the arc in the YZ plane with the pair of engagement portions 334a of the first pressing portion 331 being respectively guided by the pairs of guide portions 333a. Note that the guide portions 333a may be formed as pins and the engagement portions 334a may be formed as holes with which the guide portions 333a are engaged.

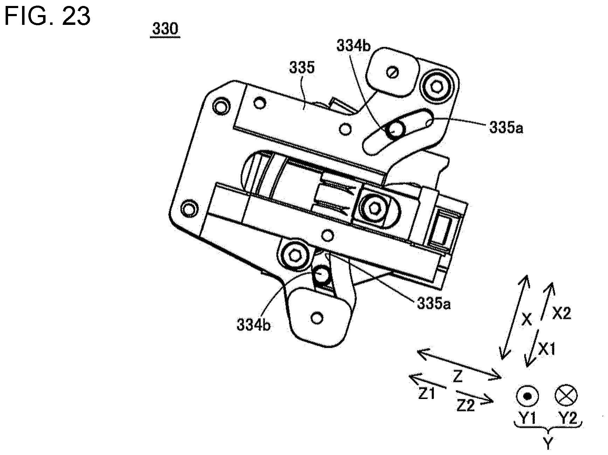

As illustrated in FIG. 23, the first driven part 330 includes a frame 335 that supports the second pressing portion 332. The frame 335 is configured such that the second pressing portion 332 is sandwiched by the frame 335 in the Y direction. The frame 335 includes guide portions 335a each of which is formed as holes extending in an arc shape. A pair of guide portions 335a among the guide portions 335a are provided, on an X2 side of the frame 335, at a Y1 side portion and a Y2 side portion, opposed to each other, of the frame 335. One of the guide portions 335a is provided, on an X1 side of the frame 335, at only the Y2 side portion of the frame 335. At an X1 side end and an X2 side end of the second pressing portion 332 include engagement portions 334b each of which is formed in a pin shape extending in the Y direction. The engagement portion 334b on the X1 side is inserted in and engaged with the one guide portion 335a provided on the X1 side of the frame 335, while the engagement portion 334b on the X2 side is inserted in and engaged with the pair of the guide portions 335a provided on the X2 side of the frame 335. The second pressing portion 332 is configured to be movable in the arc in the XZ plane orthogonal to the YZ plane, with the engagement portions 334b being respectively guided by the guide portions 333a. Note that the guide portions 335a may be formed as pins and the engagement portions 334b may be formed as holes with which the guide portions 335a are engaged.

The frame 333 and the frame 335 are formed of metal, resin, or the like.

In a first embodiment, as illustrated in FIG. 19, the first pressing portion 331 and the second pressing portion 332 are configured to be driven by elongate elements 360, respectively.

Specifically, the first pressing portion 331 is provided with a pair of grooves 331d extending in the Y direction. The elongate elements 360 are arranged in the pair of grooves 331d, respectively. One end of one of the elongate elements 360 is fixed to the first pressing portion 331 at a Y2 side end of one of the pair of grooves 331d. The other end of the one elongate element 360 arranged in the one of the pair of grooves 331d is wound around the rotation member 311 (311a, see FIG. 18), so that the first protrusion 331b of the first pressing portion 331 is moved along the arc in the Y1 direction and the Z2 direction. With this, the Y1 side (the switch portion 204a) of the first switch 204 is pressed by the first protrusion 331b of the first pressing portion 331. Further, one end of the other elongate element 360 is fixed to the first pressing portion 331 at a Y1 side end of the other groove 331d among the pair of grooves 331d. The other end of the other elongate element 360 arranged in the other groove 331d is wound around the rotation member 311 (311a, see FIG. 18), so that the second protrusion 331c of the first pressing portion 331 is moved along the arc in the Y2 direction and the Z2 direction. With this, the Y2 side (the switch portion 204b) of the first switch 204 is pressed by the second protrusion 331c of the first pressing portion 331.

The second pressing portion 332 is provided with a pair of grooves 332d extending in the X direction. The elongate elements 360 are arranged in the pair of grooves 332d, respectively. One end of one of the elongate elements 360 is fixed to the second pressing portion 332 at an X2 side end of one of the pair of grooves 332d. The other end of the one elongate element 360 arranged in the one groove 332d is wound around the rotation member 311 (311b, see FIG. 18), so that by the third protrusion 332b of the second pressing portion 332 is moved along the arc in the X1 direction and the Z2 direction. With this, the X1 side (the switch portion 204c) of the first switch 204 is pressed by the third protrusion 332b of the second pressing portion 332. Further, one end of the other elongate element 360 is fixed to the second pressing portion 332 at an X1 side end of the other of the pair of grooves 332d. The other end of the other elongate element 360 arranged in the other groove 332d is wound around the rotation member 311 (311b, see FIG. 18), so that the fourth protrusion 332c of the second pressing portion 332 is moved along the arc in the X2 direction and the Z2 direction. With this, the X2 side (the switch portion 204d) of the first switch 204 is pressed by the fourth protrusion 332c of the second pressing portion 332.

In a first embodiment, as illustrated in FIG. 19, at the initial position, a clearance CL1 or a gap is provided between the first pressing portion 331 and the second pressing portion 332 so as to avoid interference between the first pressing portion 331 and the second pressing portion 332. Specifically, at the initial position, the first and second pressing portions 331 and 332 are arranged such that the opening 331a of the first pressing portion 331 is provided between both ends of the substantially C shape of the second pressing portion 332 in the X direction. Further, the elongate elements 360 that are inserted into the pair of grooves 332d of the second pressing portion 332 penetrate the opening 331a of the first pressing portion 331. Then, when the second pressing portion 332 is moved, any one of the both ends of the substantially C-shaped of the second pressing portion 332 is moved to be arranged in the opening 331a of the first pressing portion 331 so as not to abut on the first pressing portion 331. As a result, the interference between the first pressing portion 331 and the second pressing portion 332 is avoided. Note that the clearance CL1 is an example of a "first clearance."

As illustrated in FIGS. 21 and 22, at the initial position, the second pressing portion 332 is arranged between the first protrusion 331b and the second protrusion 331c of the first pressing portion 331 and spaced away from the first pressing portion 331. Since the first pressing portion 331 moves in such a state of being separated from the second pressing portion 332, the interference between the first pressing portion 331 and the second pressing portion 332 is avoided.

Further, in a first embodiment, the first driven part 330 is configured such that, when one of the first pressing portion 331 and the second pressing portion 332 is driven, the other of the first pressing portion 331 and the second pressing portion 332 is placed at the initial position. Specifically, assuming a state where the first pressing portion 331 has moved from the initial position, the second pressing portion 332 is driven after the first pressing portion 331 returns to the initial position before the movement (the center position of the arc-shaped movement) . To the contrary, assuming a state where the second pressing portion 332 has moved from the initial position, the first pressing portion 331 is configured to be driven after the second pressing portion 332 returns to the initial position of the second pressing portion 332 (the center position of the arc-shaped movement), that is, the first pressing portion 331 is not driven until the second pressing portion 332 returns to the initial position of the second pressing portion 332.

Further, as illustrated in FIG. 19, for the first driven part 330, four pulleys 336a, 336b, 336c and 336d are provided. The elongate element 360 that is wound around the pulley 336a is inserted into one of the pair of grooves 331d of the first pressing portion 331. The elongate element 360 that is wound around the pulley 336b is inserted into the other of the pair of grooves 331d of the first pressing portion 331. The elongate element 360 that is wound around the pulley 336c is inserted into one of the pair of grooves 332d of the second pressing portion 332. The elongate element 360 that is wound around the pulley 336d is inserted into the other of the pair of grooves 332d of the second pressing portion 332.

As illustrated in FIGS. 18 and 19, the elongate element 360 that is wound around the pulley 336a and the elongate element 360 that is wound around the pulley 336b are wound around the rotation member 311 (the rotation member 311a) via pulleys 313 of the interface portion 310. As the rotation member 311a rotates about the rotation axis thereof toward one side (or the other side), the first pressing portion 331 moves along the arc. Further, the elongate element 360 that is wound around the pulley 336c and the elongate element 360 that is wound around the pulley 336d are wound around the rotation member 311 (the rotation member 311b) via pulleys 313 of the interface portion 310. As the rotation member 311b rotates about the rotation axis thereof toward one side (or the other side), the second pressing portion 332 moves along the arc.

### (Second Driven Part 340)

In a first embodiment, as illustrated in FIGS. 19 and 20, the pressing portions of the second driven part 340 includes a pair of arm portions (rocker arms) 341 that rotate about axes thereof extending in the Z direction toward the second switches 205a and 205b of the manual surgical instrument 200. The second driven part 340 includes a worm portion 342 and a worm wheel 343 arranged in the vicinity of the arm portions 341 and are configured to be driven by an elongate element(s) 360. The arm portions 341 are configured to be driven by the worm portion 342 and the worm wheel 343.

Specifically, the worm portion 342 has a substantially cylindrical shape extending in the Y direction. The worm portion 342 also serves as a capstan in which the elongate element(s) 360 is wound around the Y1 side portion and the Y2 side portion thereof.

The worm wheel 343 includes a shaft member 343a extending in the Z direction. A Z1 side portion of the shaft member 343a includes a gear member 343b that engages with a central portion of the worm portion 342 in the Y direction. A Z2 side portion of the shaft member 343a is integrally formed with a contact portion 344 capable of contacting with the arm portions 341 such that the contact portion 344 integrally rotates with the shaft member 343a. The contact portion 344 has a cam shape and is provided to extend in the radial direction of the shaft member 343a. Both longitudinal end portions of the contact portion 344 include rotatable shaft members 344a respectively. The arm portions 341 are provided corresponding to the shaft members 344a provided on the Y1 side and the Y2 side of the contact portion 344.

As illustrated in FIG. 18, the elongate element(s) 360 that is wound around the worm portion 342 is wound around the rotation member 311 (e.g., the rotation member 311c) via pulleys 313 of the interface portion 310. By rotating the rotation member 311c toward one direction around the rotation axis thereof extending in the Y direction, the worm portion 342, the worm wheel 343, and the contact portion 344 are rotated, and thus the shaft member 344a on the Y2 side of the contact portion 344 and the arm portion 341 on the Y2 side come into contact with each other. Accordingly, the arm portion 341 on the Y2 side is rotated to the press the second switch 205a. Similarly, by rotating the rotation member 311c toward the other direction around the rotation axis thereof extending in the Y direction, the worm portion 342, the worm wheel 343, and the contact portion 344 are rotated, and thus the shaft member 344a on the Y1 side of the contact portion 344 and the arm portion 341 on the Y1 side come into contact with each other. As a result, the arm portion 341 on the Y1 side is rotated to press the second switch 205b.

As illustrated in FIG. 17, the second driven part 340 is provided with a frame 345. The arm portion 341, the worm portion 342, and the worm wheel 343 are fixed to (supported by) the frame 345. The frame 345 is formed of metal, resin, or the like.

### (Third Driven Part 350)

In an embodiment, as illustrated in FIGS. 19 and 20, the pressing portion of the third driven part 350 includes an arm portion (rocker arm) 351 that moves along an operation direction of the third switch 206 of the manual surgical instrument 200. The third driven part 350 includes a worm portion 352 and a worm wheel 353 arranged in the vicinity of the arm portion 351 and are configured to be driven by an elongate element(s) 360. The arm portion 351 is configured to be driven by the worm portion 352 and the worm wheel 353.

Specifically, the worm portion 352 has a substantially cylindrical shape extending in the Y direction. The worm portion 352 also serves as a capstan in which the elongate element(s) 360 is wound around the Y1 side portion and the Y2 side portion thereof.

The worm wheel 353 includes a shaft member 353a extending in the Z direction. A Z2 side portion of the shaft member 353a includes a gear member 353b that engages with a central portion of the worm portion 352 in the Y direction. A Z1 side portion of the shaft member 353a is integrally formed with a contact portion 353c capable of contacting with the arm portion 351 such that the contact portion 353c integrally rotates with the shaft member 353a. The contact portion 353c has a cam shape and is provided to extend in the radial direction of the shaft member 353a. One of end portions of the contact portion 353c is provided with a rotatable shaft member 353d. The arm portion 351 is provided on the Y2 side so as to correspond to the shaft member 353d of the contact portion 353c.

Further, the elongate element(s) 360 that is wound around the worm portion 352 is wound around the rotation member 311 (e.g., the rotation member 311d) via pulleys 354 and pulleys 313 of the interface portion 310. By rotating the rotation member 311d toward one direction around the rotation axis thereof extending in the Y direction, the worm portion 352, the worm wheel 353, and the contact portion 353c are rotated, and thus the shaft member 353d of the contact portion 353c and the Y1 side end portion of the arm portion 351 come into contact with each other. As a result, the arm portion 351 is rotated to press the third switch 206.

As illustrated in FIG. 17, the third driven part 350 is provided with a frame 355. The arm portion 351, the worm portion 352, and the worm wheel 353 are fixed to (supported by) the frame 355. The frame 355 is formed of metal, resin, or the like.

### (Housing)

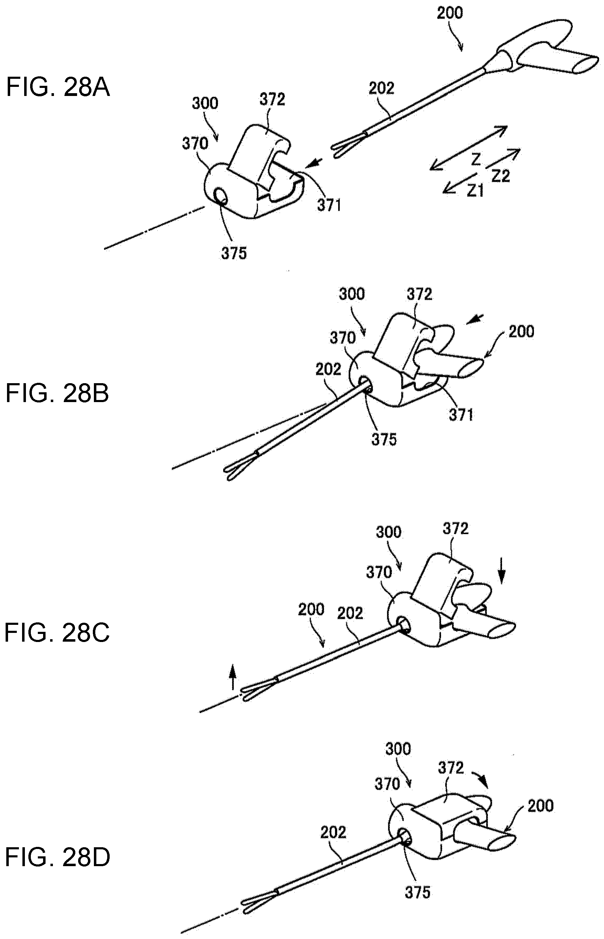

In a first embodiment, as illustrated in FIGS. 24 and 25, the surgical instrument adaptor 300 includes a housing 370 that accommodates therein the interface portion 310, the driven unit 320, the elongate elements 360, and the guide tubes 361. The housing 370 includes an opening 371 (see FIG. 28A) for inserting the manual surgical instrument 200 into the housing 370, and an opening/closing cover 372 or a lid that covers the opening 371. Further, the housing 370 includes: an opening 373 or a hole into which the grip portion 201 of the manual surgical instrument 200 is inserted; an opening 374 or a hole into which a rear end side portion 207 (the portion opposite to the shaft 202) of the manual surgical instrument 200 is inserted; and an opening 375 or a hole into which a front end side portion 208 (a proximal end side portion of the shaft 202) of the manual surgical instrument 200 is inserted. The opening 373, the opening 374, and the opening 375 have shapes corresponding to the shapes of the grip portion 201, the rear end side portion 207, and the front end side portion 208, respectively. Further, the opening 374 is provided so as to straddle the cover 372 and a portion (housing body portion 376) other than the cover 372 . Therefore, the opening 374 is configured to surround the rear end portion 207 with the cover 372 being closed. Note that the driven unit 320 (each of the driven parts 330, 340, and 350) is fixed to the inner wall of the housing 370.

Further, in a first embodiment, as illustrated in FIGS. 28A to 28D, the cover 372 opens and closes with respect to the housing 370 in such a manner that, when the cover 372 is opened, the cover 372 opens the Z2 side (an upstream side in the attachment direction (mounting direction) of the manual surgical instrument 200 to the housing 370) of the housing 370 . In other words, when the cover 372 is opened from the state where the manual surgical instrument 200 is housed in the housing 370, the rear end portion 207 of the manual surgical instrument 200 is exposed from the housing 370 with the cover 372 opened. The housing 370 is made of, for example, metal or resin.

### (Guide Tube, Reinforcement Member, Adjustment Mechanism)

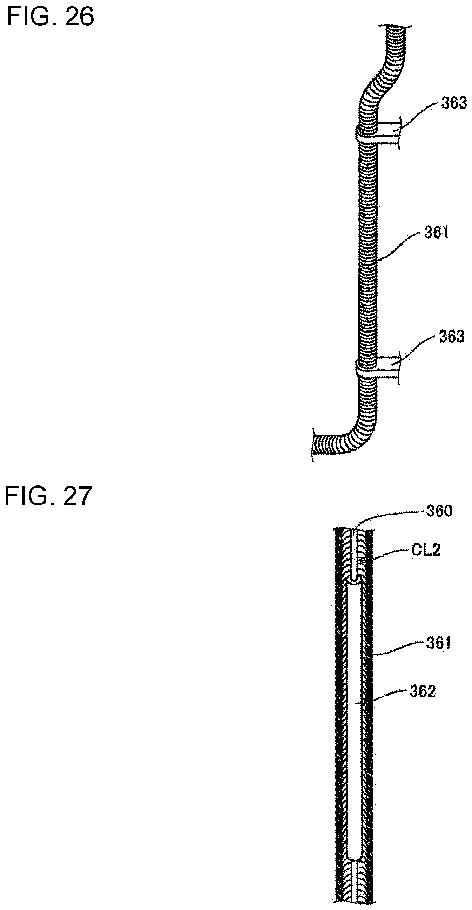

In a first embodiment, as illustrated in FIGS. 26 and 27, each of the elongate elements 360 is inserted into the guide tube 361 such that a clearance CL2 is provided between the elongate element 360 and the guide tube 361. In the clearance CL2, a reinforcement member 362 that reinforces the elongate element 360 is provided. The guide tube 361 is made of metal, resin, or the like. An inner diameter of the guide tube 361 is larger than a diameter of the elongate element 360. Accordingly, the clearance CL2 is provided between (an outer circumference of) the elongate element 360 and (an inner circumference of) the guide tube 361. The reinforcement member 362 is made of metal or the like. The reinforcement member 362 has a pipe shape, such that the elongate element 360 is inserted in the reinforcement member 362. The reinforcement member 362 is provided on a part of the elongate element 360 in the guide tube 361. Note that instead of inserting the elongate element 360 into the hollow reinforcement member 362, the reinforcement member may be formed as a solid member forming a part of the elongate element 360.

Further, in a first embodiment, the reinforcement member 362 is provided so as to reinforce a linear portion (straight portion) of the elongate element 360. In the housing 370, the elongate element 360 (the guide tube 361) is linearly supported by a pair of support members 363. The reinforcement member 362 is provided in the linear portion of the elongate element 360 between the pair of support members 363 Therefore, the reinforcement member 362 is also linearly formed.

Further, in a first embodiment, as illustrated in FIG. 17, the guide tube 361 is provided with an adjustment mechanism 364 for adjusting a length of the guide tube 361. The adjustment mechanism 364 is composed of a cable adjuster or the like in which two members are screwed together. By rotating the adjustment mechanism 364, the length of the guide tube 360 is adjusted. Accordingly, an initial tension of the elongate element 360 is adjusted.

### (Interface Portion)

In a first embodiment, as illustrated in FIG. 18, the interface portion 310 includes a base 315 including an attachment surface (mounting surface) that is attached (mounted) to the robot arm 60 via the adaptor 220. One end of the rotation member 311 in the rotation axis direction is supported by the base 315 of the interface portion 310. The other end of the rotation member 311 in the rotation axis direction is supported by a support member 314 (a retainer). Therefore, the rotation member 311 is held by both the interface portion 310 and the support member 314. The support member 314 has a plate shape. Further, the support member 314 is provided for the four rotation members 311. Further, the support member 314 is fixed by a screw or the like to the base 315 of the interface portion 310 on which the rotation members 311 are arranged. As a result, the rotation members 311 are sandwiched between the base 315 and the support member 314, and the rotation members 311 are rotatably supported by both the base 315 and the support member 314. The support member 314 is made of metal, resin, or the like.

### (Method of Attaching Manual Surgical Instrument)

With reference to FIGS. 28A to 28D, a method of attaching the manual surgical instrument 200 to the surgical instrument adaptor 300 is described below.

First, the cover 372 of the housing 370 of the surgical instrument adaptor 300 is opened, as illustrated in FIG. 28A.

Next, as illustrated in FIG. 28B, in a state where the manual surgical instrument 200 is tilted (a state in which the shaft 202 of the manual surgical instrument 200 is tilted with respect to the direction indicated by the dash-dot-dash line in FIG. 28B), the shaft 202 of the manual surgical instrument 200 is inserted into the opening 375. As a result, the manual surgical instrument 200 is inserted in the housing 370 so as to avoid interference between the manual surgical instrument 200 and the components arranged inside the housing 370 of the surgical instrument adaptor 300.

Next, as illustrated in FIG. 28C, the manual surgical instrument 200 is moved to restore the tilt of the manual surgical instrument 200. In this way, since the manual surgical instrument 200 is mounted in the housing 370 so as to avoid interference between the manual surgical instrument 200 and the components arranged inside the housing 370, the distances between the driven unit 320 and the first switch 204, the second switches 205a and 205b, and the third switch 206 of the manual surgical instrument 200 become relatively small. As a result, it is possible to shorten a winding length of the elongate element 360 required to generate a desired force for the pressing portions (the first pressing portion 331, the second pressing portion 332, the arm portion 341, and the arm portion 351).

Finally, as illustrated in FIG. 28D, the cover 372 is closed.

### (Effects of First Embodiment)

In a first embodiment, the following effects can be obtained.

As described above, a first embodiment includes: the interface portion 310 including the rotation members 311 that are configured to be rotationally driven by the driving force transmitted from the driving parts 75 provided at the robot arm 60; and the driven unit 320 including the pressing portions (the first pressing portion 331, the second pressing portion 332, the arm portion 341, the arm portion 351) that are configured to press the switches (204, 205a, 205b, and 206) of the manual surgical instrument 200. As a result, by the pressing portions of the driven unit 320 being driven (moved) by the driving forces of the driving parts 75 of the robot arm 60, the switches of the manual surgical instrument 200 can be operated. As a result, the existing manual surgical instrument 200 can be operated without using the surgical instrument dedicated for the surgery assist robot. Further, a first embodiment includes the guide tubes 361 that guide the elongate elements 360, each of which is composed of the wire or the cable, for transmitting the driving forces from the rotation members 311. Accordingly, even when the arrangement (layout) of the driven unit 320 is changed according to the positions of the switches of the manual surgical instrument 200, the elongate elements 360 are guided by the guide tubes 361 to the driven unit 320 whose arrangement has been changed. As a result, even when the arrangement (layout) of the driven unit 320 is changed, the driven unit 320 can be driven by the elongate elements 360 guided by the guide tubes 361. As a result, it is possible to flexibly respond to changes in the layout of the driven unit 320.

Further, in a first embodiment, as described above, the plural driven parts 330, 340, and 350 of the driven unit 320 each including the pressing portion(s) are provided so as to correspond to the plural switches (204, 205a, 205b and 206) of the manual surgical instrument 200, in such a manner that each of the plural driven parts 330, 340, and 350 is modularized. As a result, since each of the plural driven parts 330, 340, and 350 is modularized, even if the positions of the switches are changed depending on the type of the manual surgical instrument 200, the driven parts 330, 340, and 350 of the driven unit 320 can be easily arranged (laid out) so as to correspond to the positions of the switches of the manual surgical instrument 200.

Further, in a first embodiment, as described above, the pressing portions of the driven unit 320 include the arm portions (the arm portion 341, the arm portion 351) that move along the operating directions of the switches (205a, 205b and 206) of the manual surgical instrument 200. As a result, the moving directions (vectors) of the pressing portions of the driven unit 320 can be aligned with the operating directions of the switches of the manual surgical instrument 200. Therefore, the switches of the manual surgical instrument 200 can be appropriately pressed by the pressing portions of the driven unit 320.

Further, in a first embodiment, as described above, each of the arm portions (341, 351) is configured to be driven by the worm portion (342, 352) and the worm wheel (343, 353) disposed in the vicinity of the pressing portion, wherein the worm portion (342, 352) that is driven by the elongate element 360 and the worm wheel (343, 353) that engages with the worm portion. Therefore, by means of the worm portion and the worm wheel, a relatively large driving force for driving the arm portion is obtained by using a relatively small tension of the elongate element 360.

Further, in a first embodiment, as described above, the worm wheel (343, 353) includes the rotatable shaft member (343a, 353a) and the contact portion (344, 353c) that integrally rotates with the shaft member. The arm portion (341, 351) are configured to rotate when the contact portion (344, 353c) come into contact with the arm portion. As a result, unlike a case where the contact portion is provided separately from (spaced apart from) the worm wheel, the driven unit 320 can be downsized and the number of components of the driven unit 320 can be reduced.

Further, in a first embodiment, as described above, the switches (204, 205a, 205b and 206) of the manual surgical instrument 200 include the first switch 204 that is movable along the arc in the YY plane and along the arc in the XX plane orthogonal to the YY plane, and the pressing portions of the driven unit 320 include the first pressing portion 331 that is driven to move along the arc in the YY plane to press the first switch 204, and the second pressing portion 332 that is driven independently of the first pressing portion 331 to move along the arc in the XZ plane to press the first switch 204. As a result, the first switch 204 can be appropriately pressed by the first pressing portion 331 and the second pressing portion 332.

Further, in a first embodiment, as described above, the frames (333, 335) are provided with the guide portions (333a, 335a) formed in the arc shapes and supporting the first pressing portion 331 and the second pressing portion 332, respectively, and the first pressing portion 331 and the second pressing portion 332 respectively include the engagement portions (334a, 334b) inserted in and guided by the guide portions (333a, 335a). As a result, since the engagement portions (334a, 334b) are guided by the guide portions (333a, 335a), the first pressing portion 331 and the second pressing portion 332 are moved in such a manner that the first switch 204 is appropriately pressed by the first pressing portion 331 and the second pressing portion 332..

Further, in a first embodiment, as described above, the first pressing portion 331 and the second pressing portion 332 each have the groove (331d, 332d) in which the elongate element 360 is arranged and fixed. As a result, the first pressing portion 331 and the second pressing portion 332 are driven by the elongate elements 360 arranged in the grooves (331d, 332d). Therefore, the number of components of the driven unit 320 can be reduced.

Further, in a first embodiment, as described above, the first pressing portion 331 and the second pressing portion 332 are driven independently of each other.

The first pressing portion 331 and the second pressing portion 332 are configured such that, when one of the first pressing portion 331 and the second pressing portion 332 is driven, the other of the first pressing portion 331 and the second pressing portion 332 is positioned in the center of the arc movement. As a result, upon driving the first pressing portion 331 or the second pressing portion 332, it is possible to prevent the first pressing portion 331 and the second pressing portion 332 from interfering with each other by such a simple control.

Further, the housing 370 includes the opening 371 for attaching the manual surgical instrument 200 to the inside of the housing 370, and the cover 372 that is configured to open and close in the direction orthogonal to the attaching direction (Z1 direction) of the manual surgical instrument 200 to the housing 370 and covers the opening 371. As a result, by laterally opening the cover 372, the manual surgical instrument 200 can be easily attached to the inside of the housing 370.

Further, in a first embodiment, as described above, a part of the elongate element 360 in the guide tube 361 is provided with the reinforcement member 362 for reinforcing the elongate element 360. Accordingly, it possible to suppress damage (cutting, etc.) of the elongate element 360 due to contact between the elongate element 360 and the guide tube 361.

Here, the guide tube 361 may be formed to includes a first guide tube portion and a second guide tube portion provided with a gap from the first guide tube portion, and the reinforcement member 362 may be provided in the gap between the first guide tube portion and the second guide tube portion. In other words, the reinforcement member 362 may reinforce the elongate element 360 from the outside of the guide tube 361 instead of reinforcing the elongate element 360 from the inside the guide tube 361.

Further, in a first embodiment, as described above, the surgical instrument adaptor 300 includes the pair of support members 363 that linearly support the guide tube 361. The reinforcement member 362 reinforces the elongate element 360 in the area between the pair of support members 363. Here, in a case where a bent portion of the elongate element 360 is reinforced by the reinforcement member 362, it may be difficult to bent the elongate element 360 in accordance with rearrangement of the driven unit 320 according to the positions of the switches (204, 205a, 205b and 206) of the manual surgical instrument 200. In light of this, the reinforcement member 362 according to a first embodiment is provided to reinforce the linear portion of the elongate element 360. Accordingly, damages (cutting, etc.) of the elongate element 360 can be suppressed while facilitating the movement of the driven unit 320.

Further, in a first embodiment, as described above, the interface portion 310 includes the base 315 that is attached to the robot arm 60 and supports the one end of the rotation member 311 in the rotation axis direction of the rotation member 311, and the support member 314 that supports the other end of the rotation member 311 in the rotation axis direction. That is, both ends of the rotation member 311 are held by the base 315 and the support member 314 respectively. With this configuration, since both ends of the rotation member 311 are held by the interface portion 310 and the support member 314, respectively, it is possible to prevent the rotation axis of the rotation member 311 from tilting. As a result, the driven unit 320 can be appropriately driven by the elongate element 360.

Further, in a first embodiment, as described above, the guide tube 361 includes the adjustment mechanism 364 for adjusting the length of the guide tube 361 (the path length of the elongate element 360) between the rotation member 311 and each driven part (330, 340, 350). Thereby, the path length of the elongate element 360 can be adjusted by the adjustment mechanism 364 so that the elongate element 360 has an appropriate initial tension.

### (Second Embodiment)

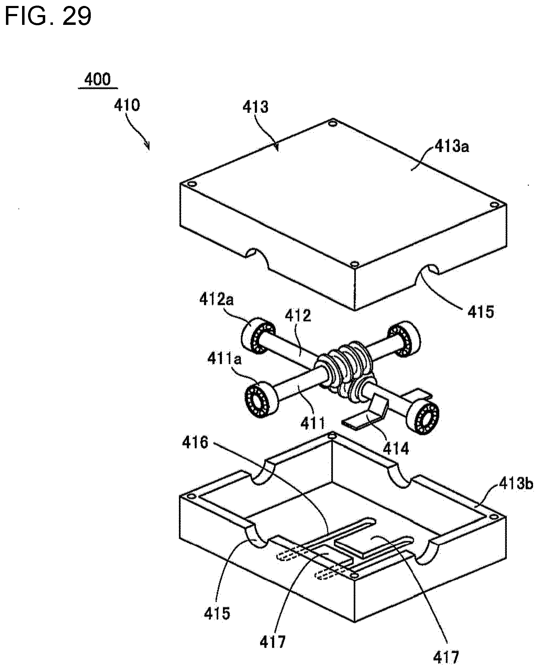

Next, a driven part 410 of the surgical instrument adaptor 400 according to a second embodiment is described with reference to FIG. 29.

The driven part 410 includes a worm 411, a worm wheel 412, and a frame 413. The worm wheel 412 is integrally provided with a contact portion 414. The contact portion 414 moves (rotates) along the operation direction of the switch (for example, the second switches 205a and 205b) of the manual surgical instrument 200.

Further, in a second embodiment, the frame 413 includes a first portion (a first frame member) 413a that covers the driven part 410 from one side and a second portion (a second frame member) 413b that is combined with the first portion 413a and covers the driven part 410 from the other side. The first portion 413a and the second portion 413b are provided with bearing support portions 415 that support bearings 411a of the worm 411 and bearings 412a of the worm wheel 412, respectively. The frame 413 is formed of metal, resin, or the like. The bearing support portions 415 are configured by notches formed in the first and second portions 413a and 413b of the frame 413..

Further, in a second embodiment, the frame 413 (the second portion 413b) includes a flexibly (resiliently) deformable arm portion 417 as a pressing portion that is integrally provided with the second portion 413b and configured to move along the operation direction of the switch of the manual surgical instrument 200. Specifically, the second portion 413b is provided with a notch 416 having a substantially H shape. A pair of arm portions 417 are defined by the substantially H-shaped notch 416 of the frame 413. The arm portions 417 move along the operation direction of the switch by the contact portion 414 rotating with the rotation of the worm wheel 412. Note that the arm portions 417 are examples of "pressing portions."

Further, in a second embodiment, the resiliently deformable arm portions 417 and the frame 413 are integrally formed of resin. That is, the frame 413 and the arm portions 417 are integrally formed by injection molding or the like.

### (Effects of Second Embodiment)

In a second embodiment, the following effects can be obtained.

In a second embodiment, as described above, the driven part 410 includes the worm 411 configured to be driven by the elongated element(s), the worm wheel 412 that engages with the worm 411, and the frame 413 that supports the worm 411 and the worm wheel 412. The frame 413 includes the resiliently deformable arm portions 417 as pressing portions that are integrally provided with the frame 413 and configured to move along the operation directions of the switch of the manual surgical instrument 200. As a result, since the arm portions 417 are integrally formed with the frame 413, the number of components of the driven part 410 can be reduced.

Further, in a second embodiment, as described above, the frame 413 and the arm portions 417 are integrally formed of resin. Thereby, the resiliently deformable arm portion 417 and the frame 413 can be easily integrally formed by resin injection molding or the like.

Further, in a second embodiment, as described above, the frame 413 includes the first portion 413a that covers the worm 411 and the worm wheel 412 from the one side, and the second portion 413b that is combined with the first portion 413a and covers the worm 411 and the worm wheel 412 from the other side. As a result, the number of members of the frame 413 is relatively small (two members which includes the first frame member 413a and the second frame member 413b), so that the configuration of the frame 413 can be simplified. Further, the frame 413 composed of the first portion 413a and the second portion 413b can be easily formed by resin injection molding or the like.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

In first and second embodiments described above, the case has been described in which the manual surgical instrument 200 is used. However, the invention is not limited thereto. For example, a manual surgical instrument having a shape other than the manual surgical instrument 200 may be used.

Further, in a first embodiment described above, the case has been described in which the three driven parts (the first driven part 330, the second driven part 340, and the third driven part 350) are provided. However, the invention is not limited thereto. The number of the driven parts may be any number other than three.

Further, in first and second embodiments described above, the case has been described in which each of the first driven part 330, the second driven part 340, and the third driven part 350 is modularized. However, the invention is not limited thereto. Each of the first driven part 330, the second driven part 340, and the third driven part 350 of the driven unit 320 may not be modularized.

Further, in first and second embodiments described above, the case has been described in which the arm portion (341, 351 and 417) is driven by the worm (342, 352, 411) and the worm wheel (343, 353, 412). However, the invention is not limited thereto. The arm portion may be driven by a mechanism other than the combination of the worm and the worm wheel.

Further, in first and second embodiments described above, the case has been described in which the contact portion (344, 353c, 414) and the worm wheel (343, 353, 412) are integrally provided with each other. However, the invention is not limited thereto. The arm portion and the worm wheel may be configured separately.

Further, in first and second embodiments described above, the case has been described in which the housing 370 includes the opening/closing cover 372. However, the invention is not limited thereto. For example, the housing may be configured to include housing members separable from each other, such that the housing members may be combined to house the manual surgical instrument 200.

In first and second embodiments described above, the case has been described in which the reinforcement member 362 for reinforcing the elongate element 360 is provided. However, the invention is not limited thereto. For example, such a reinforcement member may not be provided for the elongate element 360.

Further, in first and second embodiments described above, the case has been described in which both ends of the rotation member 311 are held by the base 315 of the interface portion 310 and the support member 314, respectively. However, the invention is not limited thereto. For example, the rotation member 311 may be cantilevered only by the base 315 of the interface portion 310.

The disclosure includes the following itemized list:
1. A surgical instrument adaptor for connecting a manual surgical instrument to a robot arm to operate the manual surgical instrument, comprising:
   an interface portion including a rotation member to be driven to rotate by a driving force transmitted from a driving part provided at the robot arm;
   a driven part including a pressing portion configured to press an operation portion of the manual surgical instrument;
   an elongate element comprising a wire or a cable connecting the rotation member and the driven part and configured to transmit the driving force from the rotation member to the driven part; and
   a guide tube that guides the elongate element.
2. The surgical instrument adaptor according to item 1, wherein
   the operation portion of the manual surgical instrument comprises a plurality of operation portions,
   the driven part comprises a plurality of driven parts provided corresponding to the plurality of operation portions, and
   each of the plurality of driven parts including the pressing portion is modularized.
3. The surgical instrument adaptor according to item 1 or 2, wherein
   the pressing portion of the driven part includes an arm portion configured to move along an operating direction of the operation portion of the manual surgical instrument.
4. The surgical instrument adaptor according to item 3, wherein
   the driven part includes a worm configured to be driven by the elongate element and a worm wheel engaged with the worm, and
   the arm portion is configured to be driven by the worm and the worm wheel.
5. The surgical instrument adaptor according to item 4, wherein
   the worm wheel includes a rotatable shaft member and a contact portion that integrally rotates with the shaft member, and
   the arm portion is configured to be rotated by the contact portion of the worm wheel coming in contact with the arm portion.
6. The surgical instrument adaptor according to item 5, wherein
   the contact portion has a cam shape.
7. The surgical instrument adaptor according to any one of items 1 to 6, wherein
   the operation portion of the manual surgical instrument comprises a cross-shaped operation portion configured to move along an arc in a first surface and along an arc in a second surface orthogonal to the first surface, and
   the pressing portion of the driven part includes:
      a first pressing portion configured to move arcuately in the first surface so as to press the cross-shaped operation portion; and
      a second pressing portion configured to move arcuately in the second surface so as to press the cross-shaped operation portion.
8. The surgical instrument adaptor according to item 7, further comprising:
   a frame including guide portions that are arcuately formed and support the first pressing portion and the second pressing portion respectively, wherein
   the first pressing portion and the second pressing portion include engagement portions which are inserted in and guided by the guide portions.
9. The surgical instrument adaptor according to item 7 or 8, wherein
   each of the first pressing portion and the second pressing portion includes a groove in which the elongate element is arranged and fixed.
10. The surgical instrument adaptor according to any one of items 7 to 9, wherein
   the first pressing portion and the second pressing portion are configured to be independently driven, and
   the first pressing portion and the second pressing portion are configured such that, when one of the first pressing portion and the second pressing portion is driven, the other of the first pressing portion and the second pressing portion is positioned in a center position of an arcuate movement thereof.
11. The surgical instrument adaptor according to any one of items 1 to 10, further comprising
   a housing that accommodates therein the interface portion, the driven part, the guide tube, wherein
   the housing includes: an opening for mounting the manual surgical instrument to an inside of the housing; and a cover that covers the opening and is configured to open and close in a direction orthogonal to a direction of mounting the manual surgical instrument to the housing.
12. The surgical instrument adaptor according to any one of items 1 to 11, wherein
   a part of the elongate element in the guide tube is provided with a reinforcement member that reinforces the elongate element.
13. The surgical instrument adaptor according to item 12, wherein
   the guide tube includes a first guide tube portion and a second guide tube portion spaced away from the first guide tube portion with a gap, and
   the reinforcement member is provided in the gap between the first guide tube portion and the second guide tube portion.
14. The surgical instrument adaptor according to item 13, further comprising
   a pair of support members that linearly support the guide tube, and
   the reinforcement member is provided to reinforce the elongate element between the pair of support members.
15. The surgical instrument adaptor according to any one of items 1 to 14, wherein
   the interface portion includes a base that is to be attached to the robot arm and supports one end of the rotation member in a rotation axis direction of the rotation member, and a support member that supports the other end of the rotation member in the rotation axis direction, such that the rotation member is held by both the base and the support member.
16. The surgical instrument adaptor according to any one of items 1 to 15, wherein
   the guide tube includes an adjustment mechanism configured to adjust a path length of the elongate element between the rotation member and the driven part.
17. The surgical instrument adaptor according to item 1, wherein
   the driven part includes a worm configured to be driven by the elongate element, a worm wheel engaged with the worm, and a frame that supports the worm and the worm wheel, and
   the frame includes a resiliently deformable arm portion that is integrally provided with the frame and configured to move along an operating direction of the operation portion of the manual surgical instrument.
18. The surgical instrument adaptor according to item 17, wherein
   the arm portion and the frame are integrally formed of resin.
19. The surgical instrument adaptor according to item 18, wherein
   the frame includes a first frame member that covers the worm and the worm wheel from one side, and a second frame member that is combined with the first frame member and covers the worm and the worm wheel from the other side.
20. A surgery assist robot comprising:
   a robot arm including a driving part; and
   the surgical instrument adaptor according to any one of items 1 to 19.

## Claims

1. A surgical instrument adaptor for connecting a manual surgical instrument to a robot arm to operate the manual surgical instrument, comprising:
an interface portion including a rotation member to be driven to rotate by a driving force transmitted from a driving part provided at the robot arm;
a driven part including a pressing portion configured to press an operation portion of the manual surgical instrument;
an elongate element comprising a wire or a cable connecting the rotation member and the driven part and configured to transmit the driving force from the rotation member to the driven part; and
a guide tube that guides the elongate element.

2. The surgical instrument adaptor according to claim 1, wherein
the operation portion of the manual surgical instrument comprises a plurality of operation portions,
the driven part comprises a plurality of driven parts provided corresponding to the plurality of operation portions, and
each of the plurality of driven parts including the pressing portion is modularized.

3. The surgical instrument adaptor according to claim 1 or 2, wherein
the pressing portion of the driven part includes an arm portion configured to move along an operating direction of the operation portion of the manual surgical instrument.

4. The surgical instrument adaptor according to claim 3, wherein
the driven part includes a worm configured to be driven by the elongate element and a worm wheel engaged with the worm, and
the arm portion is configured to be driven by the worm and the worm wheel.

5. The surgical instrument adaptor according to claim 4, wherein
the worm wheel includes a rotatable shaft member and a contact portion that integrally rotates with the shaft member, and
the arm portion is configured to be rotated by the contact portion of the worm wheel coming in contact with the arm portion.

6. The surgical instrument adaptor according to claim 5, wherein
the contact portion has a cam shape.

7. The surgical instrument adaptor according to any one of claims 1 to 6, wherein
the operation portion of the manual surgical instrument comprises a cross-shaped operation portion configured to move along an arc in a first surface and along an arc in a second surface orthogonal to the first surface, and
the pressing portion of the driven part includes:
a first pressing portion configured to move arcuately in the first surface so as to press the cross-shaped operation portion; and
a second pressing portion configured to move arcuately in the second surface so as to press the cross-shaped operation portion.

8. The surgical instrument adaptor according to claim 7, further comprising:
a frame including guide portions that are arcuately formed and support the first pressing portion and the second pressing portion respectively, wherein
the first pressing portion and the second pressing portion include engagement portions which are inserted in and guided by the guide portions.

9. The surgical instrument adaptor according to claim 7 or 8, wherein
each of the first pressing portion and the second pressing portion includes a groove in which the elongate element is arranged and fixed.

10. The surgical instrument adaptor according to any one of claims 7 to 9, wherein
the first pressing portion and the second pressing portion are configured to be independently driven, and
the first pressing portion and the second pressing portion are configured such that, when one of the first pressing portion and the second pressing portion is driven, the other of the first pressing portion and the second pressing portion is positioned in a center position of an arcuate movement thereof.

11. The surgical instrument adaptor according to any one of claims 1 to 10, further comprising
a housing that accommodates therein the interface portion, the driven part, the guide tube, wherein
the housing includes: an opening for mounting the manual surgical instrument to an inside of the housing; and a cover that covers the opening and is configured to open and close in a direction orthogonal to a direction of mounting the manual surgical instrument to the housing.

12. The surgical instrument adaptor according to any one of claims 1 to 11, wherein
a part of the elongate element in the guide tube is provided with a reinforcement member that reinforces the elongate element.

13. The surgical instrument adaptor according to claim 12, wherein
the guide tube includes a first guide tube portion and a second guide tube portion spaced away from the first guide tube portion with a gap, and
the reinforcement member is provided in the gap between the first guide tube portion and the second guide tube portion.

14. The surgical instrument adaptor according to any one of claims 1 to 13, wherein
the guide tube includes an adjustment mechanism configured to adjust a path length of the elongate element between the rotation member and the driven part.

15. A surgery assist robot comprising:
a robot arm including a driving part; and
the surgical instrument adaptor according to any one of claims 1 to 14.
